# EUROPEAN PATENT SPECIFICATION

(11) **EP 3 946 081 B1**
(45) Date of publication and mention of the grant of the patent: **03.09.2025**
(21) Application number: 20724932.7
(22) Date of filing: 01.04.2020
(51) Int. Cl.: A61B 17/10, A61B 17/00, A61B 17/04, A61B 17/06

(54) **WOUND CLOSURE**
WUNDVERSCHLUSS
FERMETURE DE PLAIE

(30) Priority: 01.04.2019 US 201962827590 P
(43) Date of publication of application: 09.02.2022
(73) Proprietor: The Provost, Fellows, Foundation Scholars and the other members of Board, of the College of the Holy and Undivided Trinity of Queen Elizabeth,, Dublin 2 (IE)
(72) Inventor: DOCTOR, Cyrus, Dublin, D04 T4A7 (IE); DAVIS, Travis Allen, Dublin, D04 F5X6 (IE)
(74) Representative: Purdylucey Intellectual Property
(86) International application number: PCT/IB2020/053109
(87) International publication number: WO 2020/202037

(56) References cited:
- WO-A1-87/04055
- WO-A2-2015/193503
- US-A- 4 006 747
- US-A- 4 347 932
- US-A- 4 417 656
- US-A- 4 471 781
- US-A1- 2001 039 426
- US-A1- 2005 251 177
- US-A1- 2009 093 824
- US-B2- 7 066 944

## Description

### FIELD

Surgical devices, systems, and methods are provided for closing wounds.

### BACKGROUND

Wound closure, for example closing lacerations or surgical incisions in skin of a patient, is often accomplished through use of sutures, surgical staples, or medical adhesives. Sutures can be both slow to apply and cause wound inversion, in which dead tissue along a top surface of skin is pushed inward toward the wound, which can slow down healing time and can cause greater scarring. Sutures and staples can lead to scarring and infection, and they can be painful during application. Medical adhesives generally can be ineffective in permanently or substantively closing a wound and are often used as dressing rather than any type of permanent closure mechanism.

US 4 471 781 A describes surgical fasteners for use with a handheld device in which the fastener is made up of a filament member terminated at each end by identical rod-shaped anchoring heads. US 7 066 944 B2 describes a fastener having an anchor at each end of a connecting portion and at least one of the anchors is expandable. However, the disclosures fail to describe successful wound eversion closures.

Therefore, improved wound closure techniques are needed.

### SUMMARY

Methods, devices, and systems are provided herein for implanting a wound closure tag for closing a wound. The invention itself is disclosed in the appended claims.

In one aspect, a system for closing a wound is provided that has at least one wound closure member with a first anchor, a second anchor, and a body extending between and coupling the first anchor and the second anchor. The wound closure member is configured to provide an eversion closure force to a wound when the first anchor is positioned on a first side of the wound, the second anchor is positioned on a second side of the wound, and the body extends through tissue of the wound. The system also has a curved delivery needle with a proximal open end, a distal end that has an ejection port thereon and that terminates in a distal point, and a channel that extends between the proximal open end and the ejection port. The channel is configured to slidably receive the first anchor. The system also has a pushrod that is slidably coupled to the curved delivery needle and configured to translate the first anchor of the wound closure member along the channel from the proximal open end to the ejection port of the delivery needle.

The system can have numerous variations. For example, the first anchor can be a crossbar, and the second anchor can be a spring member selected from a group consisting of a plurality of flat or non-flat arms, a plurality of convex or concave arms, a semi-spherical structure, a semi-circular panel extending toward the anchor, and a spiral. The second anchor can also be configured to apply an adjustable eversion closure force based on movement of the wound and the selected second anchor. In some examples, at least one wound closure member can include a plurality of wound closure members frangibly coupled to one another. The plurality of wound closure members can also be frangibly coupled to by a spine. The system can also include a handle that is coupled to a proximal end of the delivery needle and is configured to receive the push rod therethrough to guide the pushrod into the needle. The handle can have a trigger configured to slidably move the pushrod. In one example, the system can include a removable and replaceable cartridge configured to receive the at least one wound closure member therein and configured to be removably mated to the handle such that the at least one wound closure member is translatable out of the cartridge and along the channel of the delivery needle upon actuation of the pushrod. The channel of the delivery needle can also have an upward slope terminating at the ejection port. In another example, the distal point of the delivery needle can include a trocar tip.

In another aspect, a method of closing a wound in tissue is provided that includes passing a curved delivery needle through tissue on first and second sides of a wound. It also includes passing a first anchor of a wound closure member through a channel in the needle from the first side to the second side of the wound such that the first anchor exits an ejection port at a distal end of the needle on the second side and engages an exterior surface of the tissue on the second side. The method further includes retracting the deliver needle to release a second anchor of the wound closure member such that the second anchor engages an exterior surface of the tissue on the first side of the wound. A body coupled between the first anchor and the second anchor extends through the tissue and across the wound. The wound closure member also applies an eversion closure force to the tissue moving the first and second sides of the tissue toward each other, thereby closing the wound.

The method can have a variety of different embodiments. For example, passing the first anchor can include advancing a pushrod along the channel of the needle to advance the first anchor through the needle. The method can also include, prior to passing the first anchor, advancing the first anchor into a proximal end of the channel in the delivery needle such that the body extends through a slot formed in a sidewall of the needle and the second anchor is positioned external to the needle. Passing the first anchor can also include actuating a trigger coupled to a handle on a proximal end of the delivery needle to advance a pushrod along the channel of the needle from a proximal open end to the ejection port to thereby pass and eject the first anchor. In one example, the method can also include rotating a cartridge to align a third anchor of a second wound closure member with the channel in the needle. In some examples, the first anchor can be a crossbar, and the second anchor can be a spring member selected from a group consisting of a plurality of flat or non-flat arms, convex or concave arms, a semi-spherical structure, a semi-circular panel extending toward the anchor, and a spiral. Furthermore, retracting the deliver needle to release the second anchor can include the wound closure member applying an adjustable eversion closure force based on movement of the wound and the selected second anchor.

### BRIEF DESCRIPTION OF DRAWINGS

The embodiments described above will be more fully understood from the following detailed description taken in conjunction with the accompanying drawings. The drawings are not intended to be drawn to scale. For purposes of clarity, not every component may be labeled in every drawing. In the drawings:
FIG. 1 is a perspective view of one embodiment of a wound closure tag;
FIG. 2 is a cross-sectional side view of the wound closure tag of FIG. 1 implanted in tissue closing an incision;
FIG. 3 is a cross-sectional perspective view of the wound closure tag and incision of FIG. 2;
FIG. 4 is a perspective view of another embodiment of a wound closure tag;
FIG. 5 is a perspective view of another embodiment of a wound closure tag;
FIG. 6 is a perspective view of another embodiment of a wound closure tag;
FIG. 7 is a perspective view of another embodiment of a wound closure tag;
FIG. 8 is a perspective view of another embodiment of a wound closure tag;
FIG. 9 is a cross-sectional side view of the wound closure tag of FIG. 8 implanted in tissue closing an incision;
FIG. 10 is a cross-sectional side view of the wound closure tag and incision of FIG. 9;
FIG. 11A is a perspective view of another embodiment of a wound closure tag;
FIG. 11B is a side view of the wound closure tag of FIG. 11A;
FIG. 11C is a perspective view of the wound closure tag of FIG. 11A;
FIG. 12 is a perspective view of another embodiment of a wound closure tag;
FIG. 13A is an end view of another embodiment of a wound closure tag viewed from a spring member toward an anchor;
FIG. 13B is an end view of another embodiment of a wound closure tag viewed from a spring member toward an anchor;
FIG. 13C is an end view of another embodiment of a wound closure tag viewed from a spring member toward an anchor;
FIG. 13D is an end view of another embodiment of a wound closure tag viewed from a spring member toward an anchor;
FIG. 13E is an end view of another embodiment of a wound closure tag viewed from a spring member toward an anchor;
FIG. 13F is an end view of another embodiment of a wound closure tag viewed from a spring member toward an anchor;
FIG. 13G is an end view of another embodiment of a wound closure tag viewed from a spring member toward an anchor;
FIG. 13H is an end view of another embodiment of a wound closure tag viewed from a spring member toward an anchor;
FIG. 13I is an end view of another embodiment of a wound closure tag viewed from a spring member toward an anchor;
FIG. 14A is a side view of the wound closure tag of FIG. 13A;
FIG. 14B is a side view of the wound closure tag of FIG. 13B;
FIG. 14C is a side view of the wound closure tag of FIG. 13C;
FIG. 14D is a side view of the wound closure tag of FIG. 13D;
FIG. 14E is a side view of the wound closure tag of FIG. 13E;
FIG. 14F is a side view of the wound closure tag of FIG. 13F;
FIG. 14G is a side view of the wound closure tag of FIG. 13G;
FIG. 14H is a side view of the wound closure tag of FIG. 13H;
FIG. 14I is a side view of the wound closure tag of FIG. 13I;
FIG. 15A is a perspective view of another embodiment of a wound closure tag;
FIG. 15B is a perspective view of the wound closure tag of FIG. 15A;
FIG. 16A is a perspective view of one embodiment of a delivery needle;
FIG. 16B is a perspective view of the delivery needle of FIG. 16A;
FIG. 16C is a perspective view of the delivery needle of FIG. 16A with a wound closure tag therein;
FIG. 17A is a cross-sectional side view of one embodiment of implanting the wound closure tag of FIG. 1 in tissue closing an incision;
FIG. 17B is a cross-sectional side view of the wound closure tag and incision of FIG. 17A;
FIG. 17C is a cross-sectional side view of the wound closure tag and incision of FIG. 17A;
FIG. 17D is a cross-sectional side view of the wound closure tag of FIG. 1 after successful delivery to the wound of FIG. 17A;
FIG. 18A is a perspective view of another embodiment of a delivery needle with the wound closure tag of FIGS. 11A-11C;
FIG. 18B is a perspective view of the delivery needle of FIG. 16A;
FIG. 19A is a perspective view of one embodiment of a delivery device for delivering wound closure tags;
FIG. 19B is a cross-sectional side view of the delivery device of FIG. 19A;
FIG. 19C is a front-to-back view of the delivery device of FIG. 19A;
FIG. 19D is an exploded perspective view of the delivery device of FIG. 19A;
FIG. 19E is a perspective view of internal components of the delivery device of FIG. 19A;
FIG. 19F is a perspective view of a wound closure tag cartridge that can be used with the delivery device of FIG. 19A;
FIG. 19G is a perspective view of a proximal side of the cartridge of FIG. 19F;
FIG. 19H is a side view of a housing of the delivery device 19A held in a supination orientation;
FIG. 19I is a perspective view of the housing of the delivery device 19A held in a neutral orientation;
FIG. 19J is a side view of the housing of the delivery device 19A held in a pronation orientation;
FIG. 19K is a cross-sectional side view of another embodiment of a cartridge;
FIG. 20A is a perspective view of another embodiment of a delivery device for wound closure tags, such as the illustrated wound closure tag of FIG. 1;
FIG. 20B is a perspective view of the delivery device of FIG. 20A;
FIG. 21 is a perspective view of another embodiment of a delivery device for delivering wound closure tags;
FIG. 22 is a perspective view of a distal end of another embodiment of a delivery device for delivering wound closure tags;
FIG. 23 is a perspective view of a plurality of the wound closure tags of FIG. 7 connected to a spine;
FIG. 24A is a perspective view of internal components of another embodiment of a delivery device for delivering wound closure tags;
FIG. 24B is a side view of the internal components of the delivery device of FIG. 24A;
FIG. 24C is a top-down view of the internal components of the delivery device of FIG. 24A;
FIG. 24D is a perspective view of a plurality of the wound closure tags of FIGS. 11A-11C on a spine;
FIG. 25 is a perspective view of a plurality of the wound closure tags of FIG. 12 connected to a spine;
FIG. 26 is a perspective view of a distal end of another embodiment of a delivery device for delivering wound closure tags;
FIG. 27A is a perspective view of another embodiment of a delivery device for wound closure tags;
FIG. 27B is a perspective view of the delivery device of FIG. 27A;
FIG. 28A is a photograph of an incision closed using multiple wound closure tags, such as the wound closure tags of FIG. 1, on Day 0;
FIG. 28B is a photograph of the incision of FIG. 28A on Day 14;
FIG. 28C is a photograph of the incision of FIG. 28A on Day 28;
FIG. 29A is a photograph of an incision closed using sutures on Day 0;
FIG. 29B is a photograph of the incision of FIG. 29A on Day 14;
FIG. 29C is a photograph of the incision of FIG. 29A on Day 28;
FIG. 30A is a photograph of an incision closed using staples on Day 0;
FIG. 30B is a photograph of the incision of FIG. 30A on Day 14;
FIG. 30C is a photograph of the incision of FIG. 30A on Day 28;
FIG. 31A is a photograph of a stress test on sutures used to close an incision;
FIG. 31B is a photograph of the incision of FIG. 31A after the stress test;
FIG. 32A is a photograph of a stress test on the wound closure tag of FIG. 1 used to close an incision; and
FIG. 32B is a photograph of the incision of FIG. 32A after the stress test.

### DETAILED DESCRIPTION

Certain exemplary embodiments will now be described to provide an overall understanding of the principles of the structure, function, manufacture, and use of the devices and methods disclosed herein. One or more examples of these embodiments are illustrated in the accompanying drawings. Those skilled in the art will understand that the devices and methods specifically described herein and illustrated in the accompanying drawings are non-limiting exemplary embodiments and that the scope of the present invention is defined solely by the claims. The features illustrated or described in connection with one exemplary embodiment may be combined with the features of other embodiments. Such modifications and variations are intended to be included within the scope of the present invention as defined by the claims.

Further, in the present disclosure, like-named components of the embodiments generally have similar features, and thus within a particular embodiment each feature of each like-named component is not necessarily fully elaborated upon. Additionally, to the extent that linear or circular dimensions are used in the description of the disclosed systems, devices, and methods, such dimensions are not intended to limit the types of shapes that can be used in conjunction with such systems, devices, and methods. A person skilled in the art will recognize that an equivalent to such linear and circular dimensions can easily be determined for any geometric shape. Sizes and shapes of the systems and devices, and the components thereof, can depend at least on the anatomy of the subject in which the systems and devices will be used, the size and shape of components with which the systems and devices will be used, and the methods and procedures in which the systems and devices will be used.

Various exemplary methods, devices, and systems are provided for closing wounds, such as lacerations or surgical incisions, using one or more wound closure members. For example, a wound closure member is provided that has an anchor on a distal end thereof, a spring member on a proximal end thereof, and a body extending between the two. The anchor can be configured to be delivered through tissue using a needle delivery device having a curved needle that is penetrated through tissue on first and second sides of a wound. The anchor can engage tissue on the second side of the wound, and the body can trail the anchor during delivery such that it extends between the two sides of the wound. The spring member can remain on the first side of the wound and secure itself against tissue on the first side. It can provide flexibility to the wound closure member such that effective tensile or compressive force is applied to tissue on both sides of the wound to close the wound. As tissue around the wound flexes and moves, the spring member can adjust the applied tensile force to maintain generally corresponding closure force to close the wound that increases or decreases based on movement of the tissue. Additionally, the anchor and body are implanted using a curved needle such that, after implantation, the anchor and the spring member rest against outer surfaces of tissue while the body extends down into tissue in a generally semi-circular shape to connect the anchor and spring member. This generally semi-circular pathway through the wound applies an upward force on tissue along the wound while the wound closure member applies a constantly adjusting closure force, which causes wound eversion or upward puckering of the skin at the wound site. Wound eversion can promote faster healing, better blood flow, and less scarring along the wound, and the wound closure member can also avoid or decrease wound inversion, or strangulation of tissue at the wound site that leads to poor closure and scarring. The wound closure member can also allow for a more rapid closure process.

The wound closure member can be delivered to tissue using a delivery device. **In** one embodiment, the wound closure member can be delivered across tissue of a wound using a needle with a curved distal portion, a channel extending at least partially therethrough, and an ejection port at a distal end thereof. The needle can be passed through first and second sides of a wound, and the anchor of the wound closure member can be passed from the first side to the second side of the wound through the channel of the needle and out of the ejection port at the distal end thereof on the second side of the wound. The needle can assist in guiding the body of the wound closure member through tissue such that the body extends between the first and second sides of the wound, and the spring member can be left by the needle on the first side of the wound to provide a secure and flexible engagement on the first side, thereby closing the wound.

The needle can be incorporated into a variety of different delivery devices, and various activators can be used to pass the anchor of the wound closure member through the channel of the needle and out the ejection port. Various delivery devices can also be used that incorporate one or more wound closure members therein, either loaded manually into the device or configured to be deployed automatically through various cartridge delivery systems discussed below.

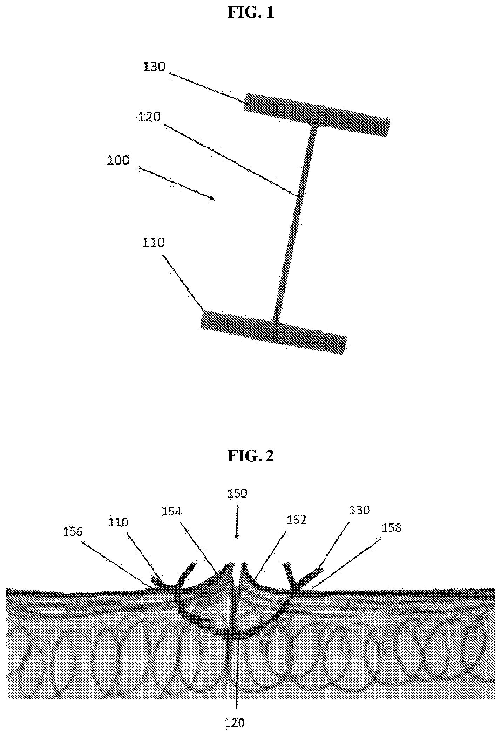

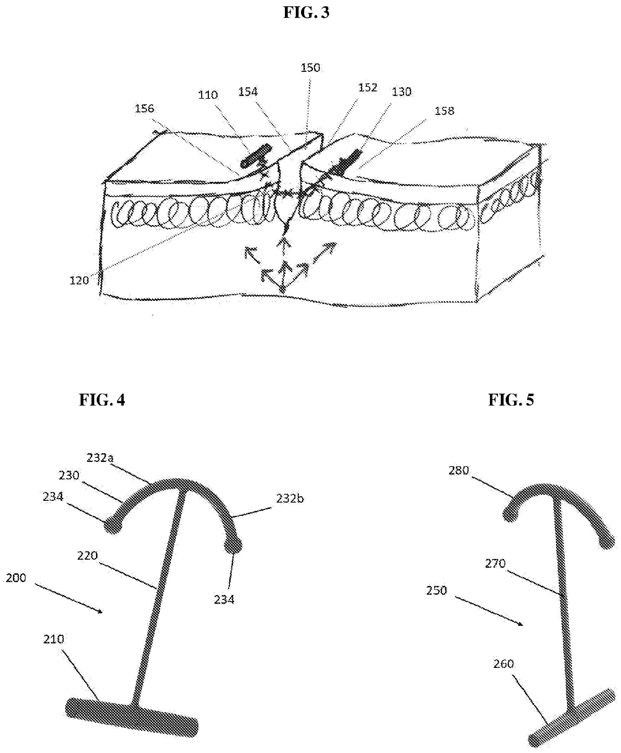

FIG. 1 illustrates one embodiment of a wound closure member in the form of a wound closure tag 100. The wound closure tag 100 has an anchor 110, an anchor or spring member 130, and a body 120 extending therebetween. The anchor 110 is shaped and sized to allow delivery through a curved needle, as discussed below, and across tissue from a first side 152 to a second side 154 of a wound 150 such that it engages and rests against an external tissue surface 156 on the second side 154 of the wound 150, as illustrated in FIG. 2. The body 120 trails behind the anchor 110 during delivery. Once implanted, it stretches between the first and second sides 152, 154 of the wound 150 to connect the anchor 110 on the second side 154 to the spring member 130 on the first side 152 and forms a generally semi-circular path through tissue. The spring member 130 is coupled to the anchor 110 through the body 120 and is designed to remain on the first side 152 of the wound such that it does not pass through tissue. Instead, it engages an external tissue surface 158 on the first side 152 of the wound 150 after delivery and acts to secure the external tissue surface 158 on the first side 152. The spring member 130 is a flexible, elastic body that flexes and bends as tissue around the wound move, thus allowing the tag 100 to apply a constantly adjusting closure force as a patient moves or shifts by allowing the spring member 130 to bend or resist movement depending on an amount of patient movement. The anchor 110, the body 120, and the spring member 130 thus operate together to apply an effective tensile closing force between the first and second sides 152, 154 of the wound 150 while not causing significant displacement of the tag 100 or significant tearing of tissue. As illustrated by the arrows in FIG. 3, the constantly adjusting tensile closing force of the tag 100 can be applied to the wound 150 along the semi-circular implant path of the body 120 that extends below a surface of the wound. As such, it can thus apply closure force that draws the first and seconds sides of the wound together while also applying an upward or pulling force, illustrated by arrows in FIG. 3, that can result in wound eversion and better healing results.

In the illustrated embodiment in FIG. 1, the wound closure tag 100 has an anchor 110 in the form of a cross-bar, a body 120 in the form of an elongate shaft or strand, and a spring member 130 in the form of a crossbar similar to anchor 110, in effect representing a second anchor. As such, anchors and spring members can represent a similar component, and a variety of other second anchors or spring members are possible, as discussed below. The body 120 extends perpendicularly between the anchor 110 and the spring member 130. The wound closure tag 100 is generally I shaped, however a variety of different shapes and orientations are possible for the spring member and/or the anchor, as discussed below. The tag 100 can be sized and shaped based on an intended surgical site to create different amounts of tensile closing force applied to the wound. For example, the tensile closing force can be varied by using a different length for the body 120 and/or using a different design or different flexibility for the spring member 130. The illustrated body 120 has a fixed length, however some bodies can have flexible or adjustable lengths that can be altered during use to allow a customized closure length and/or customized tensile closure force.

The wound closure tag 100 can be composed of a variety of materials, such as plastics, metals, medical elastomers, polymers, hydrogels, nitinol, biological tissue, absorbable, non-absorbable, etc., and various parts of the tag 100 can be comprised of different materials, such as the spring member 130 as discussed below. The tag 100 can have a variety of dimensions based on the desired application of the tag 100. However, the body 120 can have dimensions of a length of approximately 4.0 to 12.0 mm, and more preferably approximately 6.0 to 9.0 mm, and a diameter of approximately 0.05 to 0.40 mm, and more preferably approximately 0.10 to 0.35 mm. Additionally, the anchor 110 can have a length of approximately 3.0 mm to 7.0 mm, and more preferably approximately 4.0 to 6.0 mm, and a diameter of approximately 0.2 mm to 0.6 mm, and more preferably approximately 0.3 mm to 0.5 mm. The illustrated spring member 130 of FIG. 1 can have similar dimensions as anchor 100, however the spring member can have a variety of different configurations in different embodiments, as discussed below. The wound closure tag 100 can be arranged in the skin of the patient at a variety of depths and distances from the wound 150, for example the anchor 110 and the spring member 130 can extend through skin of the patient and be positioned approximately 3 to 4 mm away from the wound 150 on opposite sides thereof.

As noted, numerous different configurations of spring members can be used depending on a desired wound closure application and a desired tensile closing force. FIG. 4 illustrates another embodiment of a wound closure tag 200 similar to tag 100. Wound closure tag 200 has an anchor 210 in the form of a crossbar, a body 220 that is an elongate shaft or a strand, and a spring member 230. The spring member 230 is in the shape of a semi-circle that is coplanar with the anchor 210 and is curved toward the anchor 210. The spring member 230 has two arms 232a, 232b that each extend radially outward from the body 220 to form the semi-circular shape of the spring member 230. The terminal end of each arm has a sphere 234 that can rest against an outer tissue surface on a first side of a wound upon deployment, and the arms 232a, 232b can extend above the outer tissue surface, compressing toward the outer tissue surface when tensile closing force is required to close the wound. The curved shape of the arms 232a, 232b that form the spring member 230 can thus provide additional flex or tensile resistance to movement of the tag 200 when the tag 200 extends across first and second sides of a wound, which can also increase tensile closing force applied to tissue on both sides of the wound to more securely close the wound.

FIG. 5 illustrates a wound closure tag 250 similar to tag 200 with an anchor 260, a spring member 280, and a body 270 extending therebetween. The spring member 280 is a semi-circular member like the spring member 230, however it is in a plane perpendicular to a plane of the anchor 260, rather than being coplanar with the anchor.

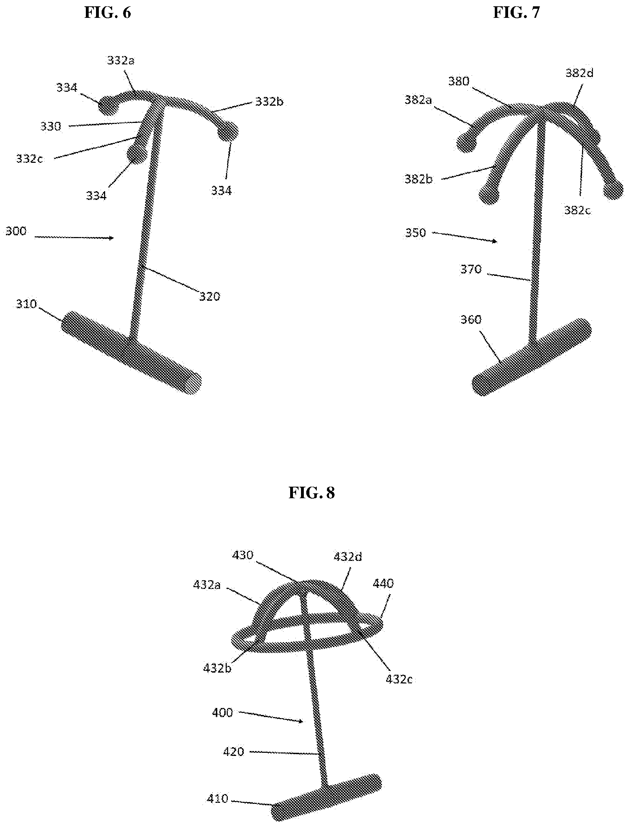

While the spring members 230, 280 each have two arms, various numbers of arms can be used and in numerous different configurations. For example, FIG. 6 illustrates another embodiment of a wound closure tag 300 similar to tags 200 and 250. The wound closure tag 300 has an anchor 310, a spring member 330, and a body 320 extending therebetween. In this embodiment, the spring member 330 has three arms 332a, 332b, 332c that each has a first end that engages the body and a second end positioned closer to the anchor 310 having a sphere 334 thereon. The spheres 334 can rest against an outer tissue surface on a first side of a wound upon deployment, and the semi-spherical shape of the spring member 330 can provide additional flex or tensile resistance to movement of the tag 300 when the tag 300, which can also create additional tensile closing force applied to both sides of the wound.

FIG. 7 illustrates a wound closure tag 350 similar to tag 300 with an anchor 360, a spring member 380, and a body 370 extending therebetween. The spring member 380 is a semi-spherical member similar to spring member 330, however it has four arms 382a, 382b, 382c, 382d curving toward the anchor 360 and defining a generally semi-spherical shape. Two arms 382b, 382d are coplanar with the anchor 360 while the other two arms 382a, 382c are in a plane perpendicular to a plane of the anchor 360. However, various other alignments of the arms are possible, such as having the arms aligned at various non-right angles with the anchor.

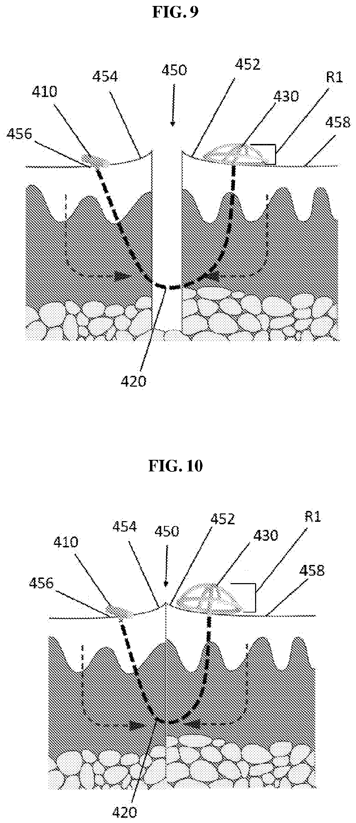

FIG. 8 illustrates another embodiment of a wound closure tag 400 similar to tags 300 and 350. The wound closure tag 400 has an anchor 410, a spring member 430, and a body 420 extending therebetween. The spring member 430 has four arms 432a, 432b, 432c, 432d that each has a first end that engages the body 420 and a second or terminal end that engages a ring 440 thereon. The ring 440 extends co-radially around the body 420 and engages all of the arms 432a, 432b, 432c, 432d so that the ring 440 and the arms 432a, 432b, 432c, 432d define a semi-spherical shape that encompasses part of the body 420 within the defined semi-sphere. When the tag 400 has been delivered from a first side 452 to a second side 454 of a wound 450, the ring 440 contacts an outer tissue surface 458 of the first side 452 to provide a large and stable contact surface for the spring member 430 against the outer tissue surface 458, as illustrated in FIGS. 9 and 10. The arms 432a, 432b, 432c, 432d can flex and bend to help the tag 400 apply tensile closing force between the two sides 452, 454 of the wound 450 by applying an upward pulling force on the body 420 when they are in a compressed state upon initial delivery. For example, the arms 432a, 432b, 432c, 432d can be compressed toward the outer tissue surface 458 upon initial delivery as the tag 400 acts to close the wound 450, as illustrated in FIG. 9. The semi-spherical shape of the spring member 430 can thus be flattened and distorted toward the outer tissue surface 458 upon delivery such that a radius R1 of the semi-spherical shape is smaller than when the spring member 430 is in a resting or pre-deployed state. As the wound 150 closes, the arms 432a, 432b, 432c, 432d can revert to a less distorted semi-spherical orientation as less tensile closing force is needed to help the tag 400 draw the two sides 452, 454 of the wound together, as illustrated in FIG. 10, allowing the radius R1 of the semi-spherical shape to increase to a value more equivalent to the radius when the spring member 430 is in a resting state.

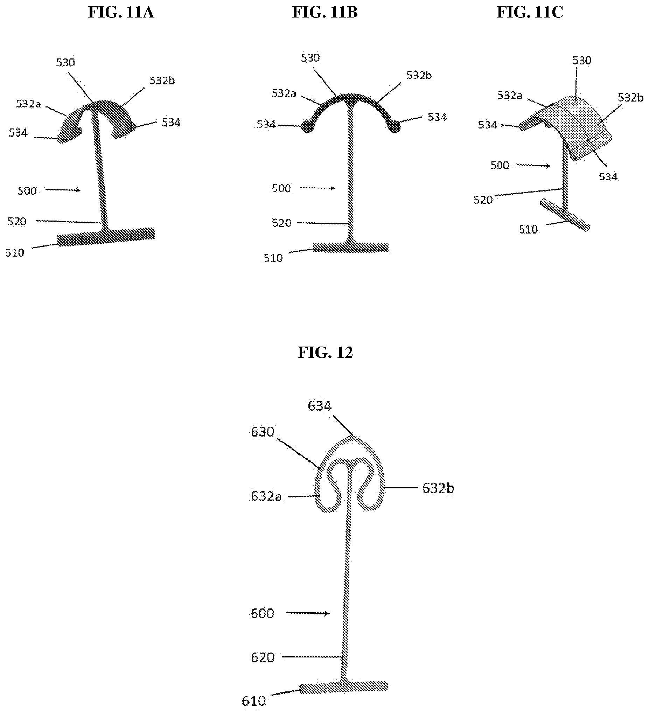

FIGS. 11A-11C illustrate another embodiment of a wound closure tag 500 similar to tags 200 and 250. The wound closure tag 500 has an anchor 510, a spring member 530, and a body 520 extending therebetween. The spring member 530 is in the shape of a semi-circular plate that extends toward the anchor 510. It has two plate arms or sides 532a, 532b that each engage the body 520 on one end and extend toward the anchor 510 on an opposite end. The arms 532a, 532b extend away from the body 520 to create the broad, semi-circular plate shape of the spring member 530, forming a generally partial cylindrical shape with the body 520 extending from an inner surface within the semi-circular curve of the partial cylinder. Each of the arms 532a, 532b terminates in a cylindrical bar 534 that extends along the edge of each arm 532a, 532b positioned closer to the anchor 510. The plate arms 532a, 532b extend away from the body 520 in a coplanar fashion to anchor 510. However, various other alignments of the plate arms 532a, 532b are possible, such as arms 532a, 532b being perpendicular to anchor 510 and/or having the arms 532a, 532b aligned at various non-right angles to the anchor. When the tag 500 is delivered to a wound and extends from a first side to a second side of the wound, the cylindrical bars 534 contact an outer tissue surface of the first side of the wound to provide a stable contact surface between the spring member 530 and the tissue, similar to ring 440 of tag 400. As the tag 500 applies tensile closing force to the wound, the arms 532a, 532b can flex and bend to help apply an increased force, similar to arms 432a, 432b, 432c, 432d. Because the spring member 530 has wide arms 532a, 532b, the force applied will be greater than a force applied by thinner arms, such as the arms on tag 200. In other embodiments, the force applied by the spring member 530 can be adjusted by varying a thickness of the plate arms 532a, 532b and/or by varying manufacturing materials and thus varying mechanical properties of the spring member 530, such as varying an elastic modulus of the spring member 530, etc. In still other embodiments, a force of spring member 530 can be adjusted by varying a geometry of spring member 530, such as by forming the spring member 530 into a semi-oval or a flexible flat plane, and thereby varying a depth and adjustable length of the body 520 and/or the spring member 530 between the cylinders 534 and the anchor 510.

FIG. 12 illustrates another embodiment of a wound closure tag 600 similar to tags 200 and 250. The wound closure tag 600 has an anchor 610, a spring member 630, and a body 620 extending therebetween. The spring member 630 is generally shaped like a heart or spade, forming a point 634 on an end opposite the anchor 610 and having two lobes 632a, 632b that each extend toward the anchor 610. However, the spring member 630 can also be rounded on the end opposite the anchor 610. Ends of the lobes 632a, 632b closest to the anchor 510 can rest against an outer tissue surface on a first side of a wound upon deployment, and the lobes 632a, 632b can each provide additional flex or tensile closing force to the anchor 600.

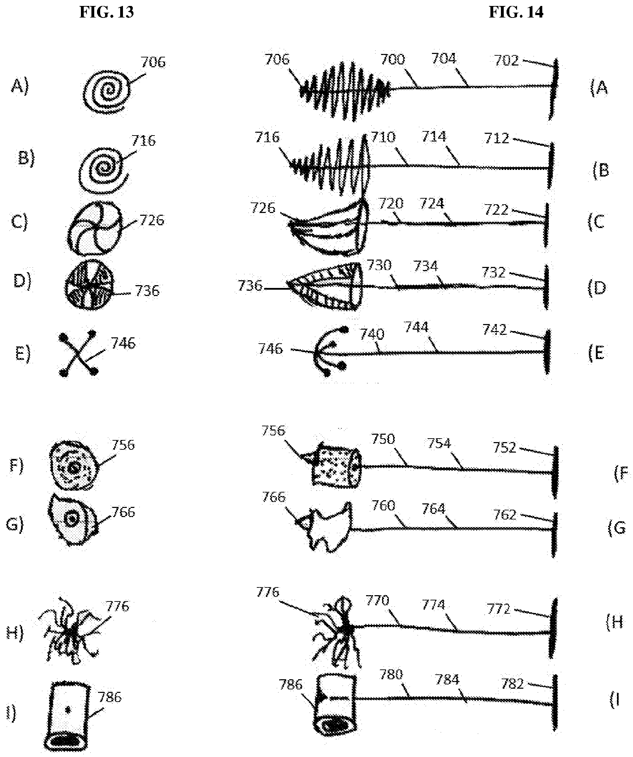

Numerous other designs can be used for the spring members, as well. For example, FIGS. 13A-13I and 14A-14I illustrate a variety of additional wound closure tags with a variety of different spring members. FIGS. 13A-13I illustrate each wound closure tag when viewed from the corresponding spring member toward the corresponding anchor, and FIGS. 14A-14I illustrate each wound closure tag when viewed from the side. For example, FIGS. 13A and 14A illustrate a wound closure tag 700 with an anchor 702, a body 704, and a spring member 706 shaped like a coiled spring or spiral that is coiled around an end of the body 704 opposite the anchor 702 and bulges along a central portion. FIGS. 13B and 14B illustrate a wound closure tag 710 with an anchor 712, a body 714, and a spring member 716 shaped like a coiled or spiraled cone with a point of the cone facing away from the anchor 712 and a wider base facing the anchor 712. FIGS. 13C and 14C illustrate a wound closure tag 720 with an anchor 722, a body 724, and a spring member 726 shaped like a wire frame of a cone with a spiraled point of the cone facing away from the anchor 722 and a wider base facing the anchor 722. FIGS. 13D and 14D illustrate a wound closure tag 730 with an anchor 732, a body 734, and a spring member 736 shaped like a cone with solid panels on the side and a point of the cone facing away from the anchor 732 with a wider base facing the anchor 732. FIGS. 13E and 14E illustrate a wound closure tag 740 with an anchor 742, a body 744, and a spring member 746 with arms similar to tag 350, but that are angularly offset from the anchor 742. FIGS. 13F and 14F illustrate a wound closure tag 750 with an anchor 752, a body 754, and a spring member 756 that is a cylinder, made from various materials such as hydrogels, elastomers, thermoplastics, partially resorbable materials, foam, nitinol, various polymers, etc. The spring member 756 can also be expandable during use. FIGS. 13G and 14G illustrate a wound closure tag 760 with an anchor 762, a body 764, and a spring member 766 that is an amorphous or expandable material, such as a biological tissue, a hydrogel, an alginate, etc. FIGS. 13H and 14H illustrate a wound closure tag 770 with an anchor 772, a body 774, and a spring member 776 that comprises a plurality of disordered arms that project from an end of the body 774 opposite the anchor 772, which results in a mop or feather duster appearance. FIGS. 13I and 14I illustrate a wound closure tag 780 with an anchor 782, a body 784, and a spring member 786 in the shape of a cylinder arranged perpendicularly to the body 784. The body 784 can slidably translate perpendicular to the centerline of the spring member 786. The cylinder of spring member 786 can be collapsible and attached to the body 784 on an external or internal face of the spring member 786 away from the anchor 782. All of these spring members can be collapsible during deployment, for example having expandable foam, frills, memory-retaining material such as nitinol kept in a compressed state of tension until deployment, etc., and they can be freeze-dried to allow deployment before they begin to expand, such as being a frozen hydrogel that begins to expand once in contact with heat and/or moisture.

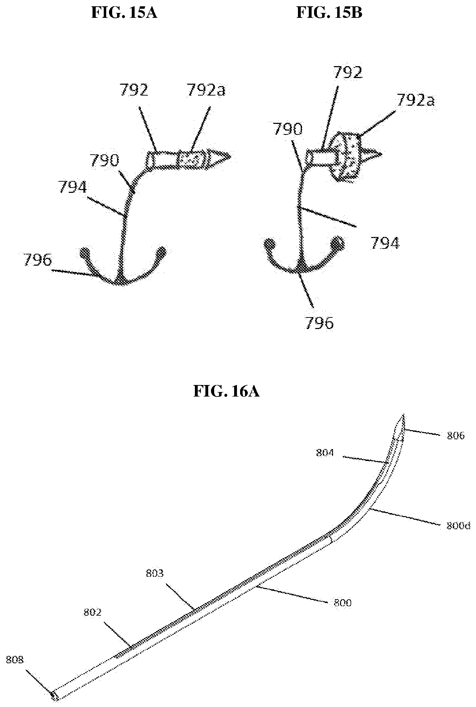

The anchor can also be collapsible during deployment and expandable once delivered. For example, FIGS. 15A and 15B illustrate a wound closure tag 790 with an anchor 792, a body 794, and a spring member 796 with arms similar to tag 200. The anchor 792 has a freeze-dried hydrogel 792a attached thereto. During deployment as illustrated in FIG. 15A, the anchor 792 is in an unexpanded state because the hydrogel 792a does not have sufficient time to react to heat and/or moisture. However, after deployment, the anchor 792 expands as the hydrogel 792a interacts with heat and/or moisture in the body, as illustrated in FIG. 15B.

The various anchors on the wound closure tags discussed above are sized and shaped to be deployed through a delivery needle. The delivery needle passes through first and second sides of a wound and, while still puncturing both sides of the wound, passes an anchor of a wound closure tag therethrough to allow the anchor to move through the first and second sides and exit the tissue on the second side to secure itself thereto. The needle also assists in guiding the body of the wound closure tag during anchor placement so that the body can extend through the first and seconds sides of the wound between the anchor and a spring member of the wound closure tag positioned on the first side of the wound.

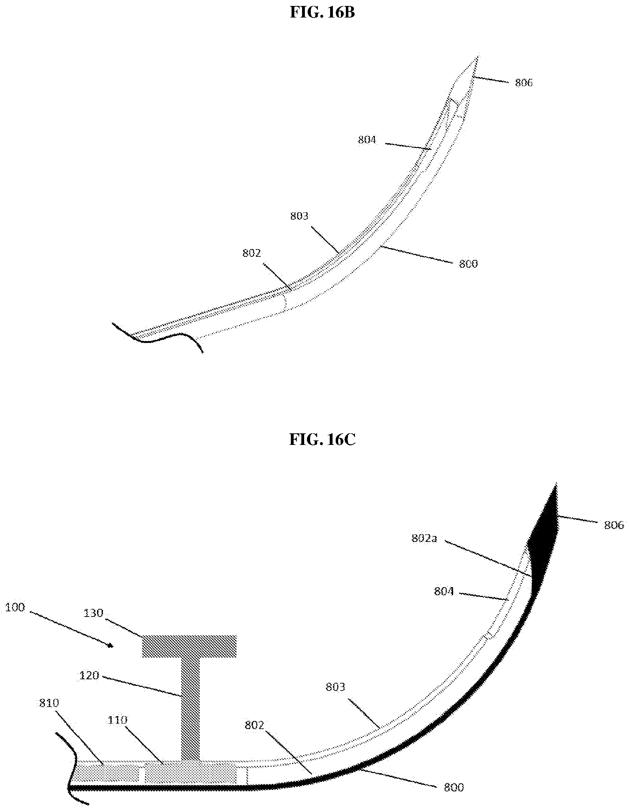

FIGS. 16A-16C illustrate an embodiment of a delivery needle 800 having a curved distal portion 800d, a channel 802 extending at least partially therealong, an ejection port 804 positioned at a distal end of the channel 802, a sharp distal point 806, and an open proximal end 808. The channel 802 is sized to receive anchors of the various wound closure tags therein such that the anchors can slide along the channel 802 during delivery, and it has a slot 803 that forms an opening or passage from the channel 802 external to the needle 800 along which the bodies can slide during delivery. The ejection port 804 at the distal end of the channel 802 allows the anchors to be ejected from the delivery device. In some embodiments, the channel 802 can have an upward slope 802a at its distal end approaching the ejection port 804 to force the anchor to exit therefrom, as illustrated in FIG. 16C with a black bar showing a general path of a bottom of the channel 802 including the upward slope 802a. The distal point 806 can be formed from a variety of types of needle tips, such as a cutting and/or trocar tip needle having broad, solid penetrating sides. An exemplary loading process is illustrated using the wound closure tag 100 in FIG. 16C, with internal interactions illustrated by broken lines, however any wound closure tag can be used. The needle 800 can allow anchors to be loaded therein by passing the anchor 110 into the ejection port 804 and sliding the anchor 110 proximally along the channel 802 with the body 120 extending from the anchor 110 through the slot 803 and the spring member 130 coupled to the body 120 and positioned external to the needle 800. The open proximal end 808 of the needle 800 can accept a flexible pushrod 810 therein so that, during delivery of the anchor to a wound site, the pushrod can push the anchor distally toward the distal tip 806 and the ejection port 804. As the flexible pushrod 810 extends through the channel 802, the needle 800 can force the anchor 110 out of the ejection port 804 as the anchor 110 slides along the slope 802a of the channel 802.

The needle 800 can be made from a variety of materials, such as medical grade metal such as steel, various rigid plastics or polymers, etc., and a variety of pushrods can be used in the form of elongate shafts and made from various materials as well, such as inconel, spring steel, polymers, nitinol, polymer braided and woven steel composites, thermoplastics, elastomers, etc.

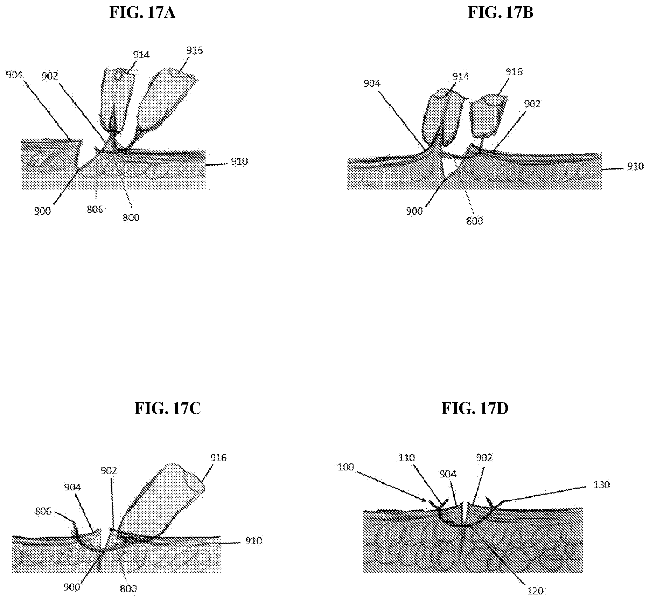

In use, once the anchor 110 has been loaded proximally into the needle 800, the needle can be passed through tissue at a wound. As illustrated in FIG. 17A, a surgeon can grip a first side 902 of a wound 900 in tissue 910 of a patient using a variety of tools, such as forceps 914. The distal point 806 of the needle 800 can then be passed through tissue on the first side 902 of the wound 900. The needle 800 illustrated in FIGS. 17A-17C is placed on a handle 916, however the needle 800 can be placed on or incorporated into a variety of devices, as discussed below. A second side 904 of the wound 900 can then be grasped, as illustrated in FIG. 17B, and the distal point 806 of the needle 800 can be passed therethrough, as illustrated in FIG. 17C. Thus, the needle 800 can extend across the wound 900 with a proximal side of the needle 800 in the first side 902 of the wound 900 and a distal side of the needle 800 in the second side 904 of the wound 900. The distal point 806 can extend out of the second side 904, and the channel 802 can thus form a passage or path through the wound 900. Since the distal portion of the needle is curved, the path will be semi-circular.

The open proximal end 808 of the needle 800 can accept a flexible pushrod 810 therein, and the anchor 110 can be pushed distally toward the distal tip 806 and the ejection port 804 upon distal advancement of the pushrod 810. The pushrod 810 can either be manually inserted and advanced by a surgeon or can be actuated through a variety of mechanisms discussed below. As the flexible pushrod 810 extends through the channel 802, the needle 800 can force the anchor out the ejection port 804 as the anchor 110 slides along the slope 802a of the channel 802, thus placing the anchor 110 on the second side 904 of the wound 900 with the body 120 extending through the two sides of the wound 900. The spring member 130 will remain on the first side 902, as illustrated in FIG. 17D. In various other embodiments, the needle can be passed through both sides of the wound simultaneously and/or forceps or other grasping instruments may not be needed depending on a comfort level of the surgeon and/or how severe or open a wound is.

Loading of one or more anchors into needles and mechanisms to actuate pushrods to pass the anchors therethrough can vary, as explained below. However, the basic process of passing a needle through each side of a wound and advancing a pushrod distally along a channel of the needle to deliver an anchor of a wound closure tag through the channel and across the wound will be used to provide delivery of the anchor and placement of the wound closure tag across the wound in the embodiments that follow.

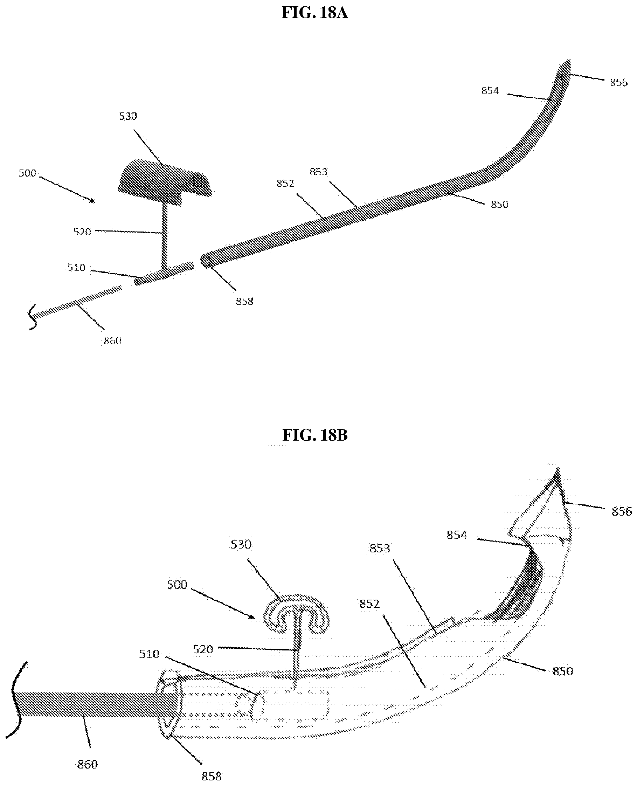

While the needle 800 is loaded manually through the ejection port 804, different loading mechanisms are possible. For example, FIGS. 18A and 18B illustrate an embodiment of a delivery needle 850 that can be loaded from a proximal end to allow delivery of multiple anchors without having to interact with a distal end of the needle. Proximal loading can allow easier loading, including automatic loading discussed below, and can avoid hassle and accidental user needle sticks caused by loading through the ejection port. The needle 850 is a curved needle that is similar to needle 800, with a channel 852 that has a slot 853 extending therealong, an ejection port 854 positioned at a distal end of the channel 852, a sharp distal point 856, and an open proximal end 858. The channel 852 extends from the open proximal end 858 to the ejection port 854. As such, the needle 850 can be loaded from the proximal open end 858.

An exemplary loading process is illustrated using the wound closure tag 500 in FIG. 18A, however any wound closure tag can be used. As illustrated, the needle 850 can accept the anchor 510 through the proximal open end 858 and into the channel 852 while the body 520 extends through the channel 852 and will slide therealong with movement of the anchor 510. The spring member 530 extends from the body 510 and will remain external to the needle. After the distal point 856 has been passed through two sides of a wound, the proximal open end 858 of the needle 850 can accept a flexible pushrod 860 that extends distally along the channel 852 to force the anchor 510 distally through the channel 852, as illustrated in FIG. 18B with broken lines representing internal engagements. The needle 850 can force the anchor 510 out the ejection port 854. As with channel 802 of needle 800, the channel 852 provides a path along which the anchor 510 can travel to move the anchor from a first side of a wound to a second side with the body 520 extending through the slot 853 and trailing behind the anchor 510 and the spring member 530 remaining on the first side. The anchor 510 is described as being loaded into the proximal open end 858 of the needle 850. However, the anchor 510 can be loaded through the ejection port 854 if desired, similar to needle 800.

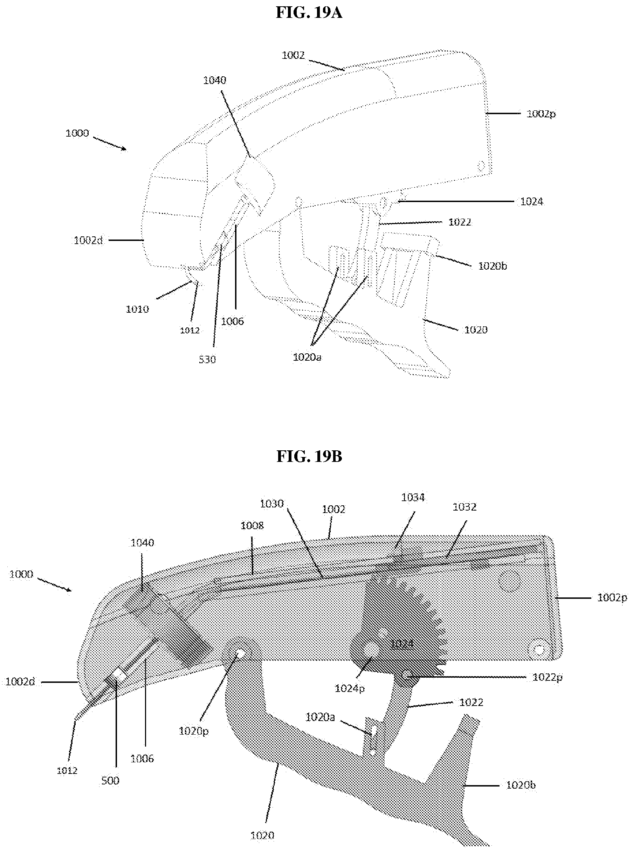

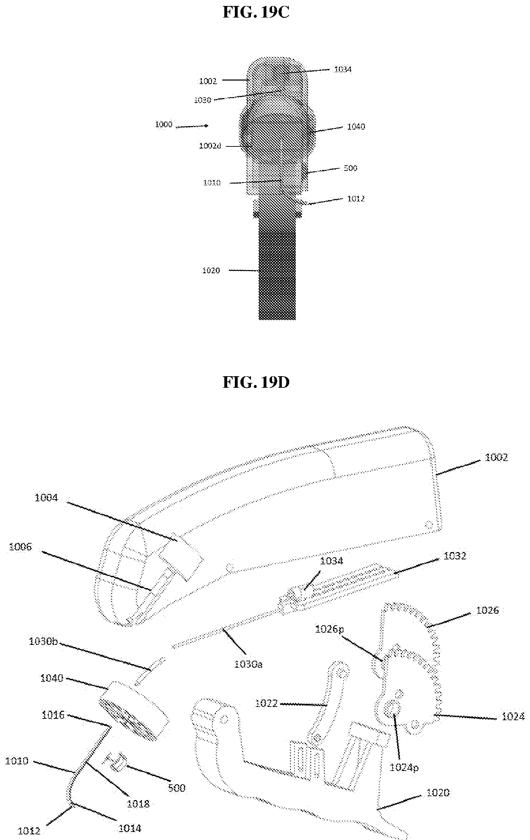

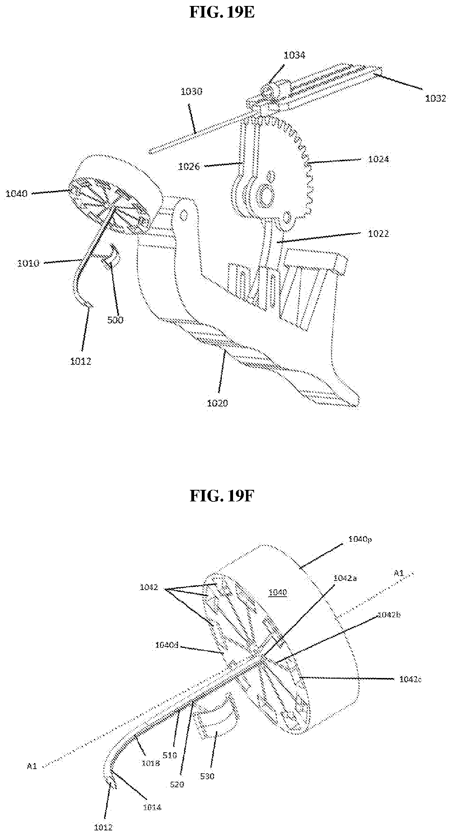

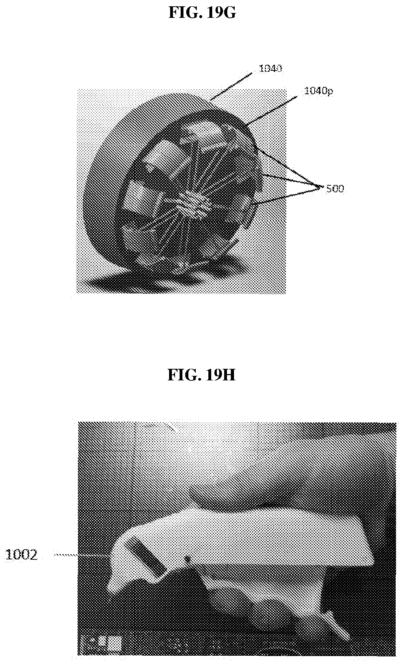

As indicated above, the needle 850 can either be manually loaded by a surgeon inserting the anchor into the needle 850 or can be loaded automatically through use of a variety of delivery devices, as discussed below. FIGS. 19A-19J illustrate one embodiment of a delivery device 1000 for delivering wound closure tags. The delivery device 1000 can allow rapid automatic loading of one or more wound closure tags during use, allowing a surgeon to quickly deliver wound closure tags to close a wound. It can also have a removable and replaceable wound closure tag cartridge that can allow a surgeon to deliver multiple wound closure tags during one operation and/or allow a surgeon to easily use different types of wound closure tags during a single operation by simply changing out the cartridge. In general, the device 1000 has a housing 1002, a curved needle 1010, an actuation mechanism with a trigger 1020, and a cartridge 1040.

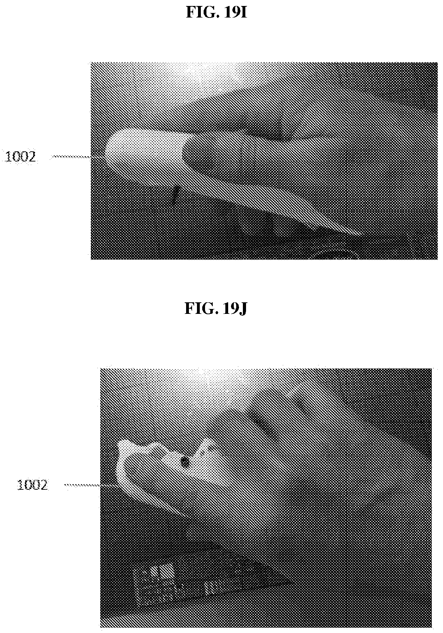

The housing 1002 is an approximately rectangular body that slopes or curves downward from a proximal end 1002p to a distal end 1002d to provide increased visibility when operating the device 1000. The housing 1002 has a cartridge opening 1004 in the form of a cavity configured to removably receive the cartridge 1040 therein and hold the cartridge 1040 in rotatable engagement through a variety of mechanisms, such as clips, protrusion and detent combinations, etc. The housing 1002 also has a body slot 1006 extending between the cartridge 1040 and a channel 1018 of the needle 1010, discussed below, to allow a body and a spring member of a wound closure tag to exit the cartridge 1040 and advance along the needle 1010 during delivery of an anchor, as discussed below. FIGS. 19A-19J show the device 1000 used with wound closure tag 500, however any wound closure tag can be used. The housing 1002 can be designed and shaped to be held similar to a needle driver to help surgeons quickly understand how to use the device, for example by being held in a supination orientation illustrated in FIG. 19H, a neutral orientation as shown in FIG. 19I, or a pronation orientation as shown in FIG. 19J.

The needle 1010 is similar to needle 850 discussed above and extends distally from the distal end 1002d of the housing 1002. It has a sharp distal point 1012, an ejection port 1014, an open proximal end 1016, and a channel 1018 extending between the two. The needle 1010 can be advanced through first and second sides of a wound and can deliver the anchor 510 of the wound closure tag 500 across the wound. The anchor 510 can be loaded from the open proximal end 1016 of the needle 1010, which is aligned with the cartridge 1040.

The cartridge 1040 contains a plurality of wound closure tags 500 therein. The cartridge 1040 can automatically load a wound closure tag 500 into the needle 1010 upon actuation of the device 1000 during use and can be rotated to align another wound closure tag 500 after delivery of the first. The illustrated cartridge 1040 is a circular-shaped body that can be received in and can rotate relative to the cartridge opening 1004, similar to the rotatable bullet cylinder in a revolver firearm, however other configurations are possible as discussed below, such as top-loading or side-loading cartridges. The cartridge 1040 has a distal side 1040d, a proximal side 1040p, and a plurality of tag openings 1042 in the form of cavities extending from the distal side 1040d to the proximal side 1040p. The tag openings 1042 are each sized and shaped to removably receive a select wound closure tag, such as the illustrated tag 500. Each tag opening 1042 receives the anchor 510 in an anchor cavity 1042a that is offset from and adjacent to a central longitudinal axis A1 of the cartridge 1040. Each anchor cavity 1042a is positioned to align with the open proximal end 1016 of the needle 1010 upon rotation of the cartridge 1040. Each opening 1042 also has a body cavity 1042b that extends radially out from the anchor cavity 1042a away from the central longitudinal axis A1 of the cartridge 1040 for receiving the body 520 of the tag 500. The body cavity 1042b terminates in a spring member cavity 1042c placed radially farthest away from the central longitudinal axis A1 of the cartridge 1040 that receives the spring member 530 therein, resulting in the cartridge 1040 being configured to receive a plurality of tags 500 that fan out from the central longitudinal axis A1. The cartridge 1040 can align one of the anchor cavities 1042a with the open proximal end 1016 of the needle 1010 and can receive a pushrod 1030 through the anchor cavity 1042a from the proximal side 1040p so that any anchor 510 contained therein is forced distally into the open proximal end 1016 of the needle 1010, thus loading the anchor 510 into the needle 1010 and causing the associated wound closure tag 500 to move distally out of the cartridge 1040 perpendicular to the central longitudinal axis A1. The anchor cavity 1042a can receive the pushrod 1030 therethrough as the tag 500 is delivered to a wound, discussed below. Once delivery is successful, the pushrod 1030 is retracted proximally out of the anchor cavity 1042a, and the cartridge 1040 can be rotated about its central longitudinal axis A1 to align the next anchor cavity 1042a with the open proximal end 1016 of the needle 1010 for optional delivery of another tag 500. In some embodiments, the cartridge 1040 can be disposable after use.

The pushrod 1030 can be part of the actuation mechanism that is used to deliver one or more wound closure tags 500 from the cartridge 1040 along the needle 1010 and across a wound. The illustrated actuation mechanism includes a trigger 1020, a lever 1022, first and second gears 1024, 1026, the pushrod 1030, a rack 1032, and an alignment member 1034. The trigger 1020 is biased to extend away from the housing 1002 and is pivotably coupled thereto at a pivot point 1020p. The trigger 1020 is coupled to the lever 1022 by a slot 1020a formed in the trigger that allows the lever 1022 to slide therealong upon actuation of the trigger 1020 by pivoting the trigger 1020 toward the housing 1002. The trigger 1020 has a stop member 1020b that contacts the housing 1002 and stops movement of the trigger 1020 upon full actuation. The lever 1022 is pivotably coupled to the first and second gears 1024, 1026 on a side opposite to the trigger 1020 at pivot point 1022p. The first and second gears 1024, 1026 are coupled to the housing 1002 at pivot points 1024p, 1026p. Actuation of the trigger 1020 causes the lever 1022 to move toward the housing 1002 and slightly proximally, causing the lever 1022 to rotate the gears 1024, 1026 about the pivot points 1024p, 1026p, thereby causing the gears 1024, 1026 to rotate toward the distal end 1002d of the housing 1002. The gears 1024, 1026 have teeth extending therefrom that engage corresponding openings in the rack 1032, which is an elongate member that extends in a slot from the proximal end 1002p of the housing 1002 toward the distal end 1002d and is distally slidable therein. The rack 1032 has the pushrod 1030 fixed to and extending from a distal end thereof, and it has an alignment member 1034 at the distal end. As the gears 1024, 1026 rotate toward the distal end 1002d of the housing 1002, the teeth thereon force the rack 1032 distally, which in turn forces the pushrod 1030 distally. If the cartridge 1040 is aligned with the pushrod 1030 and the needle 1010, the pushrod 1030 passes through the aligned anchor cavity 1042a of the cartridge 1040, through the open proximal end 1016 of the needle 1010, and into the channel 1018. Upon release of the trigger 1020, the process is reversed, moving the lever 1022 away from the housing 1002 and causing proximal rotation of the gears 1024, 1026. Upon proximal rotation, the teeth of the gears 1024, 1026 push the rack 1032 proximally in the housing 1002, which causes the pushrod 1030 to move proximally and retract proximally out of the needle 1010 and the cartridge 1040. The alignment member 1034 is configured to slide distally and proximally in the housing 1002 with distal and proximal movement of the rack 1032, and it sits in an alignment slot 1008 inside the housing 1002 that terminates at a distal point of full actuation to keep the rack 1032 and the pushrod 1030 aligned during movement and to prevent over-actuation. The pushrod 1030 can also be of a singular flexible construction or can have a flexible portion 1030a that moves slidably through a friction reducing guide 1030b, as illustrated in FIG. 19D. Furthermore, one or more springs can be provided in the housing to provide tactile feedback upon actuation and/or to slow movements of mechanisms therein. For example, a spring can be provided on the lever 1022 to provide tactile feedback and bias the device 1000 to a non-actuation state. A spring can also be provided around the alignment member 1034 to slow return of the rack 1032 after actuation.

Thus, in use, a surgeon can load the cartridge 1040 into the housing 1002 and align the cartridge 1040 with the needle 1010. The needle 1010 can be passed through two sides of a wound such that the needle 1010 extends thereacross and the distal point 1012 protrudes from the second side. The trigger 1020 can be actuated, causing rotation of the gears 1024, 1026 and subsequent distal motion of the rack 1032 and the pushrod 1030. The pushrod 1030 can extend distally through the cartridge 1040 to move an anchor 510 of a tag 500 through the open proximal end 1016 of the needle 1010 and into the channel 1018. The pushrod 1030 can continue to advance distally through the channel 1018 until the anchor 510 of the tag 500 is moved out of the ejection port 1014 and delivered to the second side of the wound, thus causing delivery of the tag 500 similar to the process discussed above. During movement of the anchor 510, the body 520 and the spring member 530 are distally advanced out of the cartridge 1040. The body 520 trails the anchor 510 along the channel 1018 of the needle 1010 and through the body slot 1006 of the housing 1002. As the anchor 510 is passed through the channel 1018 and out of the ejection port 1014 to be placed on the second side of the wound, the body 520 trails the anchor 510 through the wound to extend from the second side to the first side, and the spring member 530 will rest against the first side of the wound after being pulled free from the cartridge 1040.

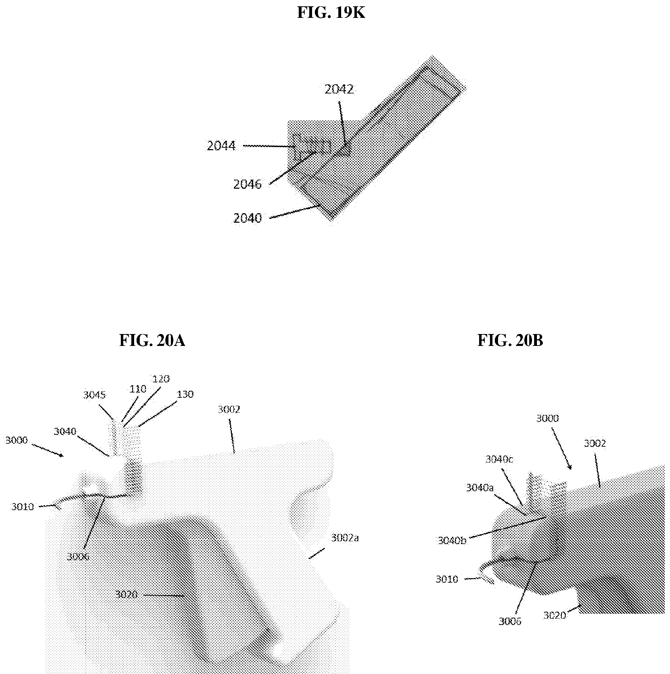

The cartridge 1040 is configured to be rotated automatically and is automatically aligned with the needle 1010. However, in various other embodiments, the cartridge can be manually rotated and/or aligned, and a variety of mechanisms can be incorporated into the cartridge, the housing 1002, or some combination of the two to assist in automatic and/or manual alignment and rotation. For example, markings on the cartridge and/or housing, ratchet mechanisms, spring and pin pairs, protrusions, detents, notches, holes, etc. can all be used. For example, FIG. 19K illustrates a cartridge 2040 similar to cartridge 1040, however it has a plurality of angled notches 2042 along a proximal side 2040p thereof. The angled notches 2042 receive a pin 2044 disposed in a housing (not shown) similar to housing 1002, and the pin 2044 is biased into engagement with the cartridge 2040 by a spring 2046. The angled notches 2042 are angled such that counterclockwise rotation of the cartridge 2040 (as viewed when facing the proximal side 2040p) causes the pin 2044 to pop out of engagement with a first notch 2042 and run along the proximal side 2040p until a second notch 2042 is reached, at which point the pin 2044 pops into engagement with the second notch 2042. This action can ensure alignment of the cartridge 2040 while also providing an audible click or pop upon alignment. The pin 2044 can also resist clockwise rotation to ensure the surgeon continues to rotate to alignment positions that have wound closure tags loaded therein until every wound closure tag has been delivered. However, other engagements are possible, and mechanisms can be incorporated into the device 1000 to automatically advance the cartridge 1040. For example, one or more pawls can be attached to the trigger 10220, the lever 1022, the gears 1024, 1026, etc. to engage a ratchet to rotate the cartridge to the next, not-yet-delivered tag 500, and another pawl can lodge in a small depression on the cartridge 1040 to stop the cartridge 1040 in a particular position so it is lined up with the needle 1010 and the pushrod 1030, similar to the rotatable bullet cylinder in a revolver firearm.

While the delivery device 10000 was described above with a rotating cartridge 1040, a variety of different delivery devices and loading mechanisms are possible, such as side or top loading and loading parallel to the pushrod. FIGS. 20A and 20B illustrate another embodiment of a delivery device 3000 that is similar to delivery device 1000, but that has top-loaded wound closure tags. The device 3000 has a housing 3002, a curved needle 3010, an actuation mechanism with a trigger 3020, and a receiving port 3040 for seating a plurality of wound closure tags. The housing 3002 is in the shape of a handgun with a pistol grip 3002a, and the needle 3010 protrudes distally therefrom. The housing 3002 has a body slot 3006 (similar to body slot 1006) through a side surface thereof and along which bodies of wound closure tags pass while the corresponding anchor is being delivered through the needle 3010. FIGS. 20A and 20B illustrate a plurality of wound closure tags 100, however any wound closure tag can be used. The needle 3010 is structured similarly to needle 1010 and is loaded through a proximal open end (not shown) in the housing 3002, and it has a channel aligned with the receiving port 3040. The trigger 3020 of the actuation mechanism is similar to trigger 1020 and is engaged with the pistol grip 3002a of the housing 3002 so that actuation of the trigger 3020 by pivoting the trigger 3020 toward the pistol grip 3002a can be achieved while holding the device 3000 by the pistol grip 3002a. Similar to the actuation mechanism of the device 1000, actuation of the trigger 3020 causes rotation of one or more gears (not shown) in the housing 3002 to drive a rack (not shown) distally to advance a pushrod (not shown) distally along a slot aligned with a bottom of the receiving port 3040 and the proximal open end of the needle 3010.

The receiving port 3040 is shaped to receive a plurality of wound closure tags 100 that are all arranged in series along a tag spine 3045 such that each anchor 110 of each tag 100 is frangibly connected to the tag spine 3045. A bottom of the receiving port in the housing 3002 is aligned at its distal end with the proximal open end of the needle 3010, and at its proximal end with the pushrod. It thus acts as an alignment surface that receives a next anchor 110 to be delivered. The receiving port 3040 has an anchor receiving channel 3040a that receives the plurality of anchors 110 connected to the spine 3045 vertically through the top of the housing 3002 so that the anchors 110 are all aligned to be loaded distally into the proximal open end of the needle 3010 when each anchor 110 reaches the bottom of the receiving port 3040. The receiving port 3040 also has a body channel 3040b that extends between the anchor receiving channel 3040a and the body slot 1006 on the housing 3002 such that the plurality of bodies 120 connected to the corresponding plurality of anchors 110 loaded into the device 3000 can move vertically downward along the body channel 3040b with downward movement of the anchors 110 and spine 3045, and then each body 120 can move distally along the body slot 3006 with its corresponding anchor 110 upon delivery through the needle 3010. The corresponding plurality of spring members 130 extend along a side of the housing 3002 and are not received therein. A spinal channel 3040c is formed vertically adjacent to the anchor receiving channel 3040a in the housing and can optionally extend entirely therethrough. The spinal channel 3040c receives the spine 3045 therealong and extends below the bottom of the anchor receiving channel 3040a. As such, a bottom portion of the spine 3045 that contained anchors 110 that have already been delivered has room to continue to move downward so that the next anchor 110 to be delivered can be kept correctly aligned with the pushrod and the needle 3010 on the bottom of the receiving port 3040 without interference from the spine 3045.

In use, the spine 3045 with the plurality of wound closure tags 100 frangibly attached thereto can be loaded into the receiving port 3040 with the anchors 110 vertically aligned in the anchor channel 3040a and the bodies 120 vertically aligned in the body channel 3040b. A wound closure tag 100 that is first in line along the spine 3045 can come to rest at the bottom of the receiving port 3040. The tag 100 can be seated in alignment with the pushrod aligned proximally behind the anchor 110 and the open end of the needle 3010 aligned distally in front of the anchor 110. After passing the needle 3010 through both sides of a wound, as discussed above, the trigger 3020 can be actuated to cause delivery of the tag 100. Upon actuation of the trigger 3020, gears and a rack inside the housing drive the pushrod distally, similar to device 1000, to pass the pushrod through the bottom of the receiving port 3040. As the pushrod passes through the receiving port, the pushrod contacts a proximal end of the first anchor 110 along the spine 3045 and forces it distally into the proximal open end of the needle 3010. The distal force of the pushrod snaps, cuts, or breaks the first anchor 110 free of the spine 3045, and the first anchor 110 is pushed distally through the needle 3010 and delivered to the second side of the wound, similar to device 1000. As the first anchor 110 is pushed distally, the first body 120 trails the anchor 110 along the body slot 3006 in the housing 3002, and the first spring member 130 is pulled distally with the body 120 but remains on the first side of the wound. Upon release of the trigger 3020, the actuation process is reversed to cause the pushrod to retract proximally out of the needle 3010 and the receiving port 3040. Once the pushrod is retracted proximally out of the receiving port 3040, a second anchor 110 of a second tag 100 in line along the spine 3045 can fall into place at the bottom of the receiving port 3040 in preparation for the actuation process to be repeated and the pushrod to break or snap off the second anchor 110 from the spine 3045 during delivery. However, in other embodiments, a variety of other tag advancement mechanisms are possible to move the next tag 100 into alignment for delivery, for example a spring member in the housing can apply downward pressure on the spine or can be engaged from underneath the spine to pull the spine down, a ratchet and/or gear mechanism can be located in the housing that is actuated upon each pull of the trigger to move the spine down a corresponding distance to align the next anchor, the plurality of tags 100 and the spine 3045 could be loaded into a top-loading vertical cartridge that contains a spring member in an upper portion thereof and provides downward force on the spine 3045, the spine 3045 can be manually moved, etc.

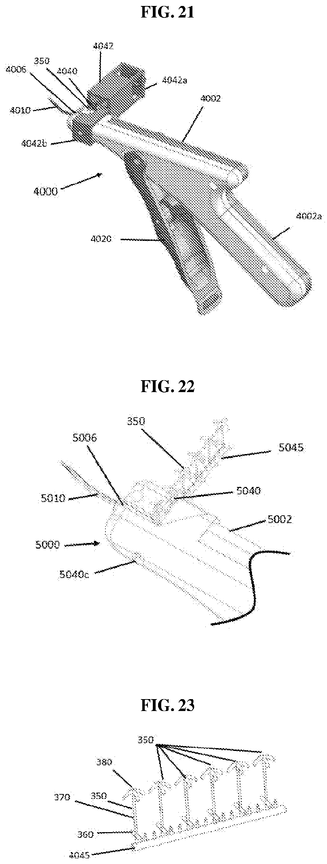

A plurality of wound closure tags can also be loaded from a side of the device in various embodiments. For example, FIG. 21 illustrates another embodiment of a delivery device 4000 for wound closure tags similar to delivery device 3000. The device 4000 has a housing 4002, a curved needle 4010, an actuation mechanism with a trigger 4020, and a receiving port 4040 for receiving a plurality of wound closure tags. The housing 4002 is in the shape of a handgun with a proximally-angled pistol grip 4002a, and the needle 4010 protrudes distally therefrom. The housing 4002 has a body slot 4006 similar to body slot 4006 and along which bodies of wound closure tags pass while the corresponding anchor is being delivered through the needle 4010. However, the body slot 4006 is in a top surface of the housing 4002 rather than a side surface. FIGS. 21-23 illustrate a plurality of wound closure tags 350, however any wound closure tag can be used. The needle 4010 is structured similarly to needle 3010 and is loaded through a proximal open end (not shown) in the housing 4002, and it has a channel aligned with the receiving port 4040. The trigger 4020 of the actuation mechanism is similar to trigger 3020 and is engaged with the pistol grip 4002a of the housing 4002 so that actuation of the trigger 4020 by pivoting the trigger 4020 toward the pistol grip 4002a can be achieved while holding the device 4000 by the pistol grip 4002a. Similar to the actuation mechanism of the device 3000, actuation of the trigger 4020 causes rotation of one or more gears (not shown) in the housing 4002 to drive a rack (not shown) distally to advance a pushrod (not shown) distally along a slot aligned with a bottom of the receiving port 4040 and the proximal open end of the needle 4010.

The receiving port 4040 is shaped to receive a plurality of wound closure tags 350 that are all arranged in series along a tag spine 4045 such that each anchor 360 of each tag 350 is frangibly connected to the tag spine 4045, as illustrated in FIG. 23. A terminal end of the receiving port 4040 along a central axis of the housing 4002 acts similarly to the bottom of the receiving port 3040 in the housing 3002 as an alignment and stopping surface for a next anchor 360 to be delivered, and it is aligned on its distal end with the proximal open end of the needle 4010 and on its proximal end with the pushrod. The receiving port 4040 also has an anchor receiving channel (not shown) that receives the plurality of anchors 360 connected to the spine 4045 horizontally through the side of the housing 4002 so that the anchors 360 are all each aligned to be loaded distally into the proximal open end of the needle 4010 when each anchor 360 reaches the terminal end of the receiving port 4040. The receiving port 4040 also has a body channel (not shown) that extends between the anchor receiving channel and the body slot 4006 on the housing 4002 such that the plurality of bodies 370 connected to the corresponding plurality of anchors 360 loaded into the device 4000 can move horizontally along the body channel and then distally along the body slot 4006 with its corresponding anchor 360 upon delivery through the needle 4010. The corresponding plurality of spring members 380 extend vertically above the housing 4002 and are not received therein. A spinal channel (not shown) is formed horizontally below the anchor receiving channel in the housing and can optionally extend entirely therethrough. The spinal channel receives the spine 4045 therealong and extends horizontally beyond the terminal end of the receiving port 4040. As such, a first portion of the spine 4045 that contained anchors 360 that have already been delivered has room to continue to move horizontally so that the next anchor 360 to be delivered can be kept correctly aligned with the pushrod and the needle 4010 at the terminal end of the receiving port 4040 without interference from the spine 4045.

The receiving port 4040 aligns with a tab housing 4042 with a holding portion 4042a and a coupling member 4042b. The coupling member 4042b extends around the housing 4002 to couple the holding portion 4042a to a side of the housing aligned with the receiving port 4040. The holding portion 4042a is a generally rectangular body that receives the plurality of tags 350 and the spine 4045 therein to provide protection to the plurality of anchors 350 and the spine 4045 during use. The holding portion 4042a can also have one or more anchor advancement mechanisms therein, such as a spring or rotatable knob at an end opposite the receiving port 4040 that can apply horizontal force onto the spine 4045 to keep a next tag 350 to be delivered in alignment with the needle 4010 and the pushrod.

In use, the device 4000 works similarly to device 3000. The spine 4045 with the plurality of wound closure tags 350 frangibly attached thereto can be loaded into the receiving port 4040, and the tab housing 4042 can be affixed to the device housing 4002. A first wound closure tag 350 that is first in line along the spine 4045 can come to rest at the terminal end of the receiving port 4040 in alignment with the pushrod proximally behind a first anchor 360 in line and in alignment with the proximal open end of the needle 4010 distally in front of the first anchor 360. After passing the needle 4010 through both sides of a wound, the trigger 4020 can be actuated to cause delivery of the tag 350. Upon actuation of the trigger 4020, gears and a rack inside the housing drive the pushrod distally to pass the pushrod through the terminal end of the receiving port 4040. The pushrod thus breaks off the first anchor 360 from the spine 4045 and forces it distally into the proximal open end of the needle 4010. The anchor 360 is forced distally through the channel of the needle 4010 and delivered to the second side of the wound with the body 370 extending across the wound and the spring member 380 remaining on the first side. Upon release of the trigger 4020, the actuation process is reversed to cause the pushrod to retract proximally out of the needle 4010 and the receiving port 4040. Once the pushrod is retracted proximally out of the receiving port 4040, a second anchor 360 of a second tag 350 in line along the spine 4045 can move horizontally into place. As referenced above, such movement can be caused by a spring or rotatable knob in the holding portion 4042a that applies horizontal force to the spine 4045. However, other mechanisms are possible in other embodiments, such as a spring member in the housing, a ratchet and/or gear mechanism can be located in the housing that is actuated upon each pull of the trigger, the spine 4045 can be manually moved, etc.

FIG. 22 illustrates another embodiment of a delivery device 5000 similar to device 4000 with a side loading mechanism. The device 5000 has a housing 5002, a curved needle 5010, an actuation mechanism with a trigger (not illustrated), a receiving port 5040 for a plurality of wound closure tags, and a body slot 5006. However, the device 5000 does not have a tab housing 4042 thereon. A spinal channel 5040c similar to spinal channel of the device 4000 extends entirely horizontally through the device 5000 such that it is open on a side opposite to the receiving port 5040. The spinal channel 5040c receives the spine 4045 coupled to the plurality of tags 350 therethrough so that a first portion of the spine 4045 that has already had its anchors 360 removed therefrom can extend horizontally away from the device 5000 during use.

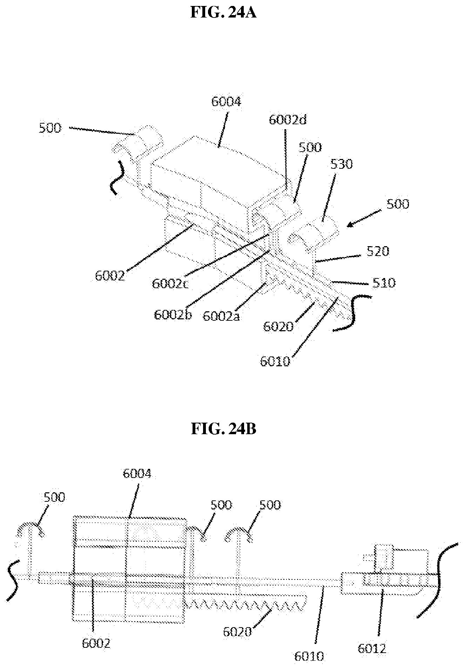

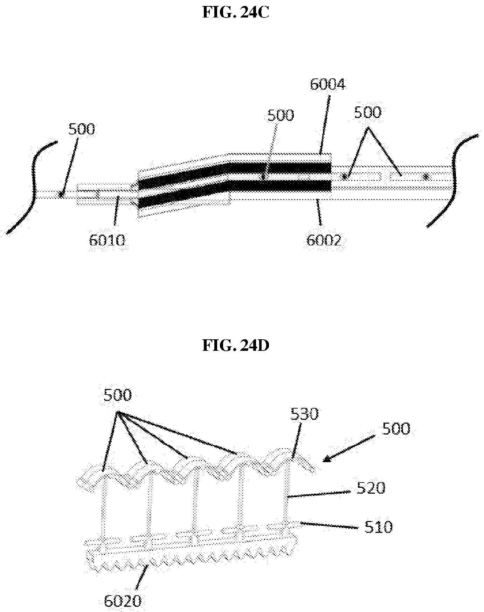

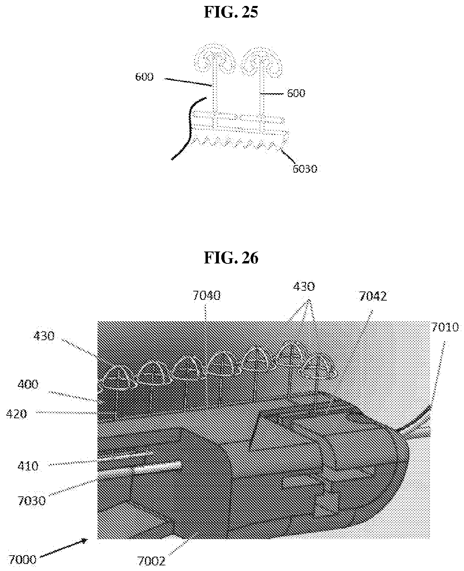

The devices 3000, 4000, 5000 have wound closure tags loaded in a perpendicular direction to movement of the corresponding pushrod. However, a plurality of wound closure tags can be loaded in a direction parallel with a pushrod of a device. FIGS. 24A-24C illustrate a loading and delivery portion of another embodiment of a delivery device for wound closure tags similar to devices 3000, 4000, and 5000 discussed above. However, instead of having a side or top loading mechanism, the loading and delivery portion illustrated in FIGS. 24A-24C has two channels extending parallel to each other, namely a pushrod channel 6002 and a wound closure tag channel 6004. The pushrod channel 6002 receives a pushrod 6010 therethrough upon actuation of the device, and the wound closure tag channel 6004 receives a plurality of wound closure tags 500 therethrough frangibly connected linearly onto a geared spine 6020. While tags 500 are illustrated in FIGS. 24A-24D, any wound closure tag can be used, such as the wound closure tag 600 on a spine 6030 in FIG. 25. The wound closure tag channel 6004 has a spine channel portion 6002a, an anchor channel portion 6002b, a body channel portion 6002c, and a spring member channel portion 6002d that each receive the corresponding spine 6020, anchors 510, bodies 520, and spring members 530 therein, respectively, when the spine 6020 and the plurality of tags 500 are loaded into the device. The spine 6020 and the plurality of tags 500 can be inserted into the wound closure tag channel 6004 from a proximal end of the device by sliding them in a distal direction. The pushrod channel 6002 and the wound closure tag channel 6004 initially extend in a direction parallel with each other and side-by-side in the housing of the device from a proximal portion of the device toward a distal end, as illustrated in FIGS. 24B and 24C. However, as highlighted by black bars in FIG. 24C, the wound closure tag channel 6004 shifts into alignment with and merges into the pushrod channel 6002 at a point that is proximal to a needle of the device but distal to the pushrod 6010 itself. As such, the wound closure tag channel 6004 effectively terminates into the pushrod channel 6002. At the point where the wound closure tag channel 6004 merges into the pushrod channel 6002, the spine 6020 is advanced distally so that the anchor 510 of the next tag 500 to be delivered in series is advanced into the merged portion of the pushrod channel 6002 proximal to the needle and distal to the pushrod 6010 while any subsequent tags 500 remain slightly proximal to the point of merger in the wound closure tag channel 6004. As such, the anchor 510 of the next tag 500 to be delivered rests alone in the merged portion of the pushrod channel 6002 with the body 520 and the spring member 530 extending vertically above the merged portion of the pushrod channel 6002. Upon actuation of the device using any of the mechanisms discussed above, such as by distal advancement of a rack 6012 coupled to the pushrod 6010, the pushrod 6010 advances distally through the pushrod channel 6002, including the merged portion of the channel in which the next tag 500 is positioned. The anchor 510 of the to-be-delivered tag 500 is snapped, cut, or broken from the spine 6020, and the pushrod 6010 forces the anchor 510 distally into a channel of the needle and out a distal side for delivery to a second side of a wound. The pushrod 6010 is then retracted proximally out of the merged portion of the pushrod channel 6002, and the spine 6020 is advanced again to load the next tag 500 into the merged portion of the pushrod channel 6002. While a merged portion of the pushrod channel 6002 has been discussed herein, the merged portion can also be the proximal open end of the needle such that the needle couples directly to the pushrod channel 6002 and the wound closure tag channel 6004 at the point of merger. The spine 6020 is advanced distally in a controlled manner through engagement of gear(s) with teeth arranged along a bottom surface of the spine 6020. The gear(s) can be rotated automatically upon actuation of the device. However, a variety of other advancement mechanisms can be used, such as manual advancement, spring mechanisms, rotation and/or actuation of various levers, knobs, buttons, release switches, etc.

In other embodiments, a spine can be excluded and the anchors of the plurality of wound closure tags can be frangibly coupled or individually arranged directly thereto. For example, FIG. 26 illustrates a delivery device 7000 similar to the devices discussed above. The device 7000 has a housing 7002, a curved needle 7010, a receiving port 7040 that extends from a proximal end to a distal end of the housing 7002, and an actuation mechanism having a pushrod 7030. A plurality of wound closure tags 430 are provided that are frangibly coupled to each other through their anchors 410 being engaged end-to-end in a linear series. While tags 400 are illustrated herein, any wound closure tags could be used. The plurality of wound closure tags 400 are loaded into the receiving port 7040 on a proximal end thereof, and they are arranged distally in series through the housing 7002 while waiting to be deployed. The anchors 410 are held in the housing 7002 with the bodies 420 extending through the housing 7002 along the receiving port 7040 and the spring members 430 extend vertically above the housing 7002. An initial tag 400 to be delivered is forced horizontally out of the receiving port 7040, breaking or separating the corresponding anchor 410 from the remaining anchors 410, and into a delivery channel 7042. As such, the anchor 410 of the corresponding tag 400 is aligned in the delivery channel 7042 distally to the pushrod 7030 and proximally to a proximal open end of the needle 7010. Upon actuation of the device 7000, similar to the actuation mechanisms discussed above, the pushrod 7030 is driven distally forward through the delivery channel 7042, through the proximal open end, and along a channel of the needle 7010. The pushrod 7030 encounters and forces the anchor 410 of the tag 400 distally for delivery, and the anchor 410 of the tag is subsequently delivered across a wound to a second side. The body 420 of the delivered tag 400 passes through the delivery channel 7042 and trails the anchor 410 across the wound, and the spring member 430 of the delivered tag 400 moves distally with movement of the anchor 410 but remains on a first side of the wound. A variety of different mechanisms can be used to break and move the to-be-delivered tag 400 horizontally into the delivery channel 7042, such as gear and push member interactions triggered by actuation of the device, spring mechanisms, manual force, etc.

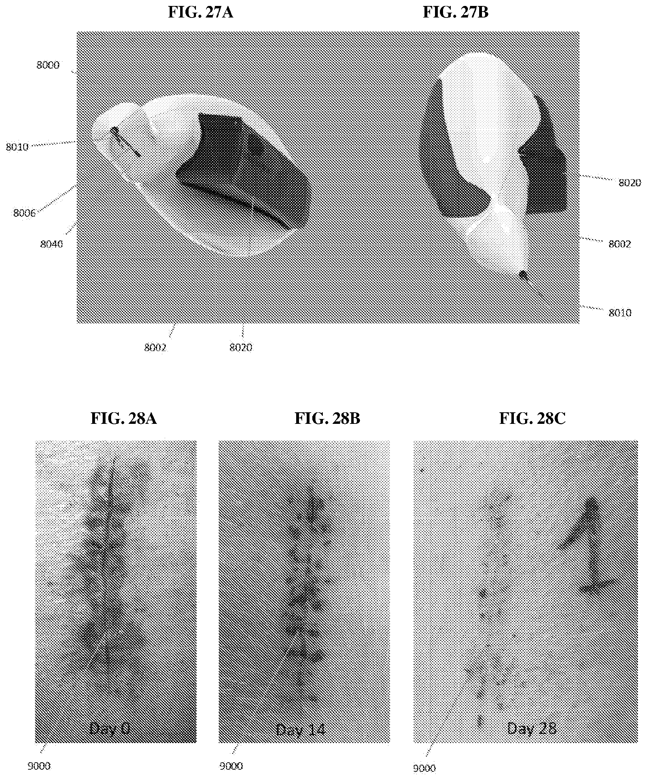

Some delivery devices can also use a mechanical actuation mechanism similar to the devices discussed above while using manual loading. For example, FIGS. 27A and 27B illustrate a delivery device 8000 similar to device 1000 for delivering wound closure tags. The device 8000 has a housing 8002, a curved needle 8010, an actuation mechanism with a trigger 8020, a tag loading port 8040, and a body slot 8006 in the housing 8002. An anchor of a wound closure tag, such as one of the wound closure tags discussed above, is inserted into the tag loading port 8040. Inside the tag loading port 8040, the anchor is aligned in a position proximal to the open end of the needle 8010 and in a position distal to a pushrod. After the needle 8010 has been passed through two sides of a wound, the trigger 8020 can be actuated to cause distal advancement of the pushrod through similar mechanisms to those discussed above. The pushrod can force the anchor distally into and through the needle 8010, resulting in delivery of the anchor on a second side of the wound. A corresponding body of the tag slides along the body slot 8006 as it trails the anchor being delivered, and a corresponding spring member is moved distally with delivery of the anchor but remains on the first side of the wound. A second tag can be loaded into the device 8000. Tags can also optionally be loaded through an ejection port on the needle 8010, as discussed above in detail.

While triggers have been illustrated for actuation and various manual, gravity-fed, or spring or rotational mechanisms have been discussed for wound closure tag advancement, a variety of different actuation and advancement mechanisms can be used in other embodiments, such as slider releases and/or push button(s) on the housing. Removable cartridges containing a plurality of wound closure tags connected, stacked, and/or arranged in series can also be used that can clip into and be subsequently released from the device using various clips, slider releases, push buttons, etc. Furthermore, while various spines are shown, other mechanisms to index and deploy wound closure tags can be used, such as frangibly connecting anchors directly together, frangibly connecting spring members directly together, or some combination of the two, etc. Various device embodiments discussed herein can also generally mimic needle drivers in ergonomic shape, manner of use, and overall design to allow surgeons to feel comfortable when first using the various delivery devices.

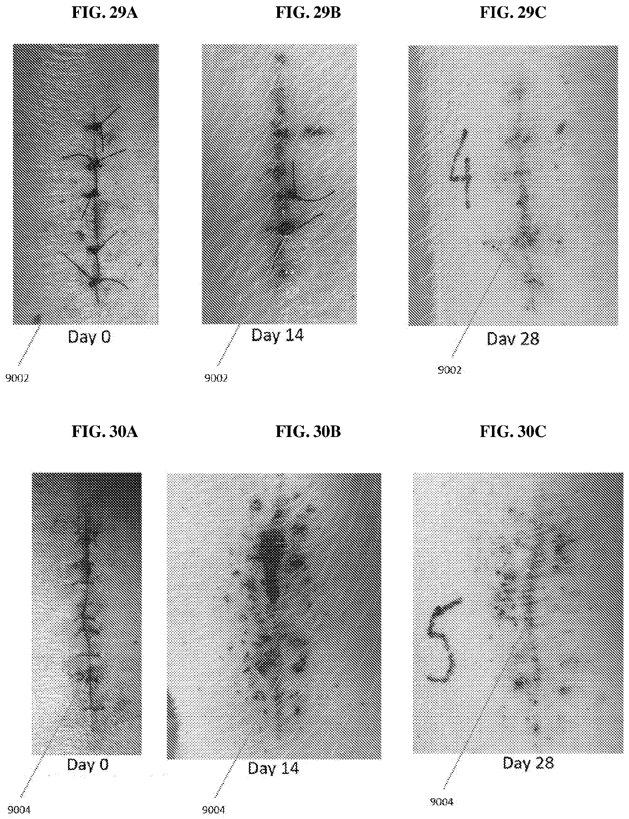

In practice, the wound closure tags discussed herein have been shown to provide faster and more effective healing and better stress displacement than sutures or staples. For example, FIGS. 28A-28C illustrate a wound 9000 closed using the wound closure tag 100 discussed above at Day 0, at Day 14, and at Day 28, respectively. The wound 9000 shows significant healing to the point of the line of incision being almost entirely gone by Day 28. A similar wound 9002 was closed using sutures, and FIGS. 29A-29C illustrate healing at Day 0, at Day 14, and at Day 28, respectively. The wound 9002 has closed by Day 28, but the incision line is still visible. Finally, another similar wound 9004 was closed using staples, and FIGS. 30A-30C illustrate healing at Day 0, at Day 14, and at Day 28, respectively. The wound 9004 still shows indications that the incision is in the process of fully closing at Day 28. Accordingly, the wound closure tag provides improved healing as compared to sutures and staples.

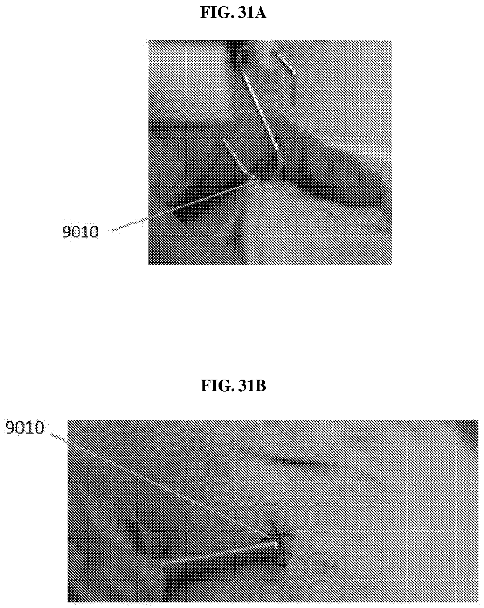

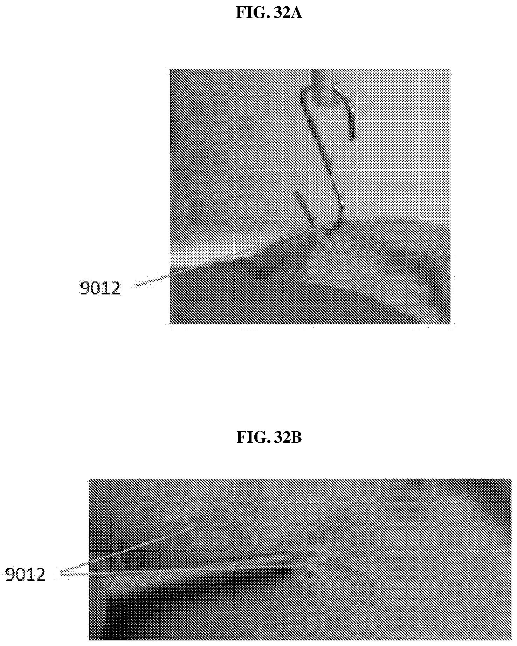

Furthermore, FIGS. 31A and 31B illustrate a tensile stress or pull test applied to a suture 9010 used to close a wound in tissue. The suture 9010 caused tearing and splitting of the skin caused by uneven distribution of force through and across the wound, as can be seen in FIG. 31B. A second tensile stress or pull test was conducted on a wound closure tag 9012, similar to tag 100 discussed herein, as illustrated in FIGS. 32A and 32B. The tag 9012 caused significantly less tearing and splitting of the skin caused by the greater distribution of force through the wound, as can be seen in FIG. 32B.

All of the devices disclosed herein can be designed to be disposed of after a single use, or they can be designed to be used multiple times. In either case, however, the devices can be reconditioned for reuse after at least one use. Reconditioning can include any combination of the steps of disassembly of the devices, followed by cleaning or replacement of particular pieces, and subsequent reassembly. In particular, the devices can be disassembled, and any number of the particular pieces or parts of the device can be selectively replaced or removed in any combination. Upon cleaning and/or replacement of particular parts, the devices can be reassembled for subsequent use either at a reconditioning facility, or by a surgical team immediately prior to a surgical procedure. Those skilled in the art will appreciate that reconditioning of a device can utilize a variety of techniques for disassembly, cleaning/replacement, and reassembly. Use of such techniques, and the resulting reconditioned device, are all within the scope of the present application.

It is preferred that devices disclosed herein be sterilized before use. This can be done by any number of ways known to those skilled in the art including beta or gamma radiation, ethylene oxide, steam, and a liquid bath (e.g., cold soak). It is preferred that device, if implanted, is hermetically sealed. This can be done by any number of ways known to those skilled in the art.

Further, in the present disclosure, like-named components of the embodiments generally have similar features, and thus within a particular embodiment each feature of each like-named component is not necessarily fully elaborated upon. Additionally, to the extent that linear or circular dimensions are used in the description of the disclosed systems, devices, and methods, such dimensions are not intended to limit the types of shapes that can be used in conjunction with such systems, devices, and methods. A person skilled in the art will recognize that an equivalent to such linear and circular dimensions can easily be determined for any geometric shape. Sizes and shapes of the systems and devices, and the components thereof, can depend at least on the anatomy of the subject in which the systems and devices will be used, the size and shape of components with which the systems and devices will be used, and the methods and procedures in which the systems and devices will be used.

## Claims

1. A system for closing a wound, comprising:
at least one wound closure member (100) having a first anchor (110), a spring member (130), and a body (120) extending between and coupling the first anchor (110) and the spring member (130);
a curved delivery needle (800) having a proximal open end (808), a distal end having an ejection port (804) thereon and terminating in a distal point (806), and a channel (802) extending between the proximal open end (808) and the ejection port (804), the channel (802) being configured to slidably receive the anchor (110); and
a pushrod (810) slidably coupled to the curved delivery needle (800) and configured to translate the first anchor (110) of the wound closure member (100) along the channel (802) from the proximal open end (808) to the ejection port (804) of the delivery needle (800);
**characterised in that** the spring member (130) is a flexible elastic body co-operable with the first anchor (110) and the body (120) to apply an effective tensile closing force between a first and second side (152,154) of the wound (150) and is configured to apply an adjustable eversion closure force to the wound (150) based on movement of the wound (150) and a shape of the spring member (130) when the spring member (150) is positioned on the second side (154) of the wound (150), the first anchor (110) is positioned on the first side (152) of the wound (150), and the body (120) extends through tissue of the wound (150).

2. The system of claim 1, wherein the first anchor (110) is a crossbar, and the shape of the spring member (130, 706, 716, 726, 736, 746, 756, 766, 776, 786) is selected from a group consisting of a plurality of non-flat arms, a plurality of convex or concave arms, a semi-spherical structure, a semi-circular panel extending toward the anchor, and a spiral.

3. The system of claim 2, wherein each selected shape of the spring member (130, 706, 716, 726, 736, 746, 756, 766, 776, 786) is configured to apply a different adjustable eversion closure force on the wound.

4. The system of claim 1, wherein the at least one wound closure member (100) comprises a plurality of wound closure members (100) frangibly coupled to one another.

5. The system of claim 4, wherein the plurality of wound closure members (100) are frangibly coupled to by a spine (3045).

6. The system of claim 1, further comprising a handle (916) coupled to a proximal end (808) of the delivery needle (800) and configured to receive the push rod (810) therethrough to guide the pushrod (810) into the needle (800), the handle (916) having a trigger (1020) configured to slidably move the pushrod (810).

7. The system of claim 6, further comprising a removable and replaceable cartridge (1040) configured to receive the at least one wound closure member (100) therein and configured to be removably mated to the handle (916) such that the at least one wound closure member (100) is translatable out of the cartridge (1040) and along the channel (802) of the delivery needle (800)upon actuation of the pushrod (810).

8. The system of claim 1, wherein the channel (802) of the delivery needle (800) has an upward slope (802a) terminating at the ejection port (804).

9. The system of claim 1, wherein the distal point (806) of the delivery needle (800) comprises a cutting tip (806).

## Patentansprüche

1. System zum Verschließen einer Wunde, umfassend:
mindestens ein Wundverschlusselement (100) mit einem ersten Anker (110), einem Federelement (130) und einem Körper (120), der sich zwischen dem ersten Anker (110) und dem Federelement (110) erstreckt und diese verbindet,
eine gekrümmte Abgabenadel (800) mit einem proximalen offenen Ende (808), einem distalen Ende, das einen Ausstoßanschluss (804) daran aufweist und in einem distalen Punkt (806) endet, und einem Kanal (802), der sich zwischen dem proximalen offenen Ende (808) und dem Ausstoßanschluss (804) erstreckt, wobei der Kanal (802) dafür gestaltet ist, den Anker (110) gleitfähig aufzunehmen, und
einen Schubstab (810), der gleitfähig mit der gekrümmten Abgabenadel (800) verbunden und dafür gestaltet ist, den ersten Anker (110) des Wundverschlusselements (100) entlang des Kanals (802) von dem proximalen offenen Ende (808) zu dem Ausstoßanschluss (804) der Abgabenadel (800) zu verschieben,
**dadurch gekennzeichnet, dass** das Federelement (130) ein flexibler elastischer Körper ist, der mit dem ersten Anker (110) und dem Körper (120) zusammenwirken kann, um eine wirksame Verschlusszugkraft zwischen einer ersten und einer zweiten Seite (152, 154) der Wunde (150) auszuüben, und dafür gestaltet ist, eine justierbare Eversionsverschlusskraft auf die Wunde (150) basierend auf einer Bewegung der Wunde (150) und einer Form des Federelements (130) auszuüben, wenn das Federelement (150) an der zweiten Seite (154) der Wunde (150) positioniert ist, der erste Anker (110) an der ersten Seite (152) der Wunde (150) positioniert ist und sich der Körper (120) durch Gewebe der Wunde (150) erstreckt.

2. System nach Anspruch 1, wobei der erste Anker (110) eine Querstange ist und die Form des Federelements (130, 706, 716, 726, 736, 746, 756, 766, 776, 786) aus einer Gruppe ausgewählt ist, die aus mehreren nicht flachen Armen, mehreren konvexen oder konkaven Armen, einer Halbkugelstruktur, einer Halbkreisplatte, die sich hin zum Anker erstreckt, und einer Spirale besteht.

3. System nach Anspruch 2, wobei jede ausgewählte Form des Federelements (130, 706, 716, 726, 736, 746, 756, 766, 776, 786) dafür gestaltet ist, eine andere justierbare Eversionsverschlusskraft auf die Wunde auszuüben.

4. System nach Anspruch 1, wobei das mindestens eine Wundverschlusselement (100) mehrere Wundverschlusselemente (100) umfasst, die zerbrechlich miteinander verbunden sind.

5. System nach Anspruch 4, wobei die mehreren Wundverschlusselemente (100) zerbrechlich durch ein Rückenteil (3045) verbunden sind.

6. System nach Anspruch 1, ferner umfassend einen Griff (916), der mit einem proximalen Ende (808) der Abgabenadel (800) verbunden und dafür gestaltet ist, den Schubstab (810) durch sich hindurch aufzunehmen, um den Schubstab (810) in die Nadel (800) zu führen, wobei der Griff (916) einen Auslöser (1020) aufweist, der dafür gestaltet ist, den Schubstab (810) gleitfähig zu bewegen.

7. System nach Anspruch 6, ferner umfassend eine abnehmbare und austauschbare Patrone (1040), die dafür gestaltet ist, das mindestens eine Wundverschlusselement (100) in sich aufzunehmen, und dafür gestaltet ist, abnehmbar mit dem Griff (916) zusammengebracht zu sein, so dass das mindestens eine Wundverschlusselement (100) auf das Betätigen des Schubstabes (810) hin aus der Patrone (1040) und entlang des Kanals (802) der Abgabenadel (800) verschiebbar ist.

8. System nach Anspruch 1, wobei der Kanal (802) der Abgabenadel (800) eine Aufwärtsneigung (802a) aufweist, die an dem Ausstoßanschluss (804) endet.

9. System nach Anspruch 1, wobei der distale Punkt (806) der Abgabenadel (800) eine Schneidespitze (806) umfasst.

## Revendications

1. Système destiné à fermer une plaie, comprenant :
au moins un élément de fermeture de plaie (100) ayant une première ancre (110), un élément de ressort (130), et un corps (120) s'étendant entre et couplant la première ancre (110) et l'élément de ressort (130) ;
une aiguille de délivrance incurvée (800) ayant une extrémité ouverte proximale (808), une extrémité distale ayant un orifice d'éjection (804) sur celle-ci et se terminant en un point distal (806), et un canal (802) s'étendant entre l'extrémité ouverte proximale (808) et l'orifice d'éjection (804), le canal (802) étant configuré pour recevoir l'ancre (110) de manière coulissante ; et
une tige de poussée (810) couplée de manière coulissante à l'aiguille de délivrance incurvée (800) et configurée pour effectuer une translation de la première ancre (110) de l'élément de fermeture de plaie (100) le long du canal (802) de l'extrémité ouverte proximale (808) à l'orifice d'éjection (804) de l'aiguille de délivrance (800) ;
**caractérisé en ce que** l'élément de ressort (130) est un corps élastique flexible capable de coopérer avec la première ancre (110) et le corps (120) pour appliquer une force de fermeture par traction efficace entre un premier et un deuxième côté (152, 154) de la plaie (150) et est configuré pour appliquer une force de fermeture par éversion ajustable à la plaie (150) sur la base du mouvement de la plaie (150) et d'une forme de l'élément de ressort (130) lorsque l'élément de ressort (150) est positionné sur le deuxième côté (154) de la plaie (150), la première ancre (110) est positionnée sur le premier côté (152) de la plaie (150), et le corps (120) s'étend à travers le tissu de la plaie (150).

2. Système selon la revendication 1, dans lequel la première ancre (110) est une barre transversale, et la forme de l'élément de ressort (130, 706, 716, 726, 736, 746, 756, 766, 776, 786) est choisie parmi un groupe constitué par une pluralité de bras non plats, une pluralité de bras convexes ou concaves, une structure d'élément semi-sphérique, un panneau semi-circulaire s'étendant vers l'ancre, et une spirale.

3. Système selon la revendication 2, dans lequel chaque forme sélectionnée de l'élément de ressort (130, 706, 716, 726, 736, 746, 756, 766, 776, 786) est configurée pour appliquer une force de fermeture par éversion ajustable différente sur la plaie.

4. Système selon la revendication 1, dans lequel l'au moins un élément de fermeture de plaie (100) comprend une pluralité d'éléments de fermeture de plaie (100) couplés de manière frangible les uns aux autres.

5. Système selon la revendication 4, dans lequel la pluralité d'éléments de fermeture de plaie (100) sont couplés de manière frangible à une épine dorsale (3045).

6. Système selon la revendication 1, comprenant en outre une poignée (916) couplée à une extrémité proximale (808) de l'aiguille de délivrance (800) et configurée pour recevoir la tige de poussée (810) à travers celle-ci pour guider la tige de poussée (810) jusque dans l'aiguille (800), la poignée (916) ayant une gâchette (1020) configurée pour déplacer de manière coulissante la tige de poussée (810).

7. Système selon la revendication 6, comprenant en outre une cartouche amovible et remplaçable (1040) configurée pour recevoir l'au moins un élément de fermeture de plaie (100) dans celle-ci et configurée pour être accouplée de manière amovible à la poignée (916) de sorte que l'au moins un élément de fermeture de plaie (100) est capable de translation hors de la cartouche (1040) et le long du canal (802) de l'aiguille de délivrance (800) lors de l'actionnement de la tige de poussée (810).

8. Système selon la revendication 1, dans lequel le canal (802) de l'aiguille de délivrance (800) a une pente ascendante (802a) se terminant au niveau de l'orifice d'éjection (804).

9. Système selon la revendication 1, dans lequel le point distal (806) de l'aiguille de délivrance (800) comprend une pointe coupante (806).
